(19) Europäisches Patentamt
European Patent Office
Office européen des brevets

(11) **EP 4 205 768 A1**

(12) **EUROPEAN PATENT APPLICATION**

(43) Date of publication:
05.07.2023 Bulletin 2023/27

(21) Application number: 22161429.0

(22) Date of filing: 10.03.2022

(51) International Patent Classification (IPC):
*A61K 47/69* (2017.01)     *A61K 41/00* (2020.01)
*A61P 35/00* (2006.01)

(52) Cooperative Patent Classification (CPC):
**A61K 47/6923; A61K 41/0052; A61K 47/6929;
A61P 35/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.12.2021 PT 2021117718**

(71) Applicant: **International Iberian Nanotechnology
Laboratory
(INL)
4715-330 Braga (PT)**

(72) Inventors:
• **GALLO PÁRAMO, JUAN**
  **4715-330 Braga (PT)**
• **BELMONTE RECHE, EFRES**
  **4715-330 Braga (PT)**
• **BAÑOBRE LÓPEZ, MANUEL**
  **4715-330 Braga (PT)**

(74) Representative: **Patentree
Edificio Net
Rua de Salazares, 842
4149-002 Porto (PT)**

(54) **MAGNETIC SOLID LIPID NANOPARTICLES, METHODS AND USES THEREOF**

(57)     A magnetic solid lipid particle comprising:
an encapsulated core, wherein said core comprises thermal effectors and MRI contrast enhancers, a surfactant for stabilizing the magnetic solid lipid particle in aqueous solution, and an active ingredient;
wherein the thermal effectors and MRI contrast enhancers are magnetic nanoparticles; a first modified phospholipid comprising a poly-ethylene glycol molecule - PEG - bonded onto the surface of the encapsulated core and; wherein said PEG modified phospholipid is temperature responsive; and
a second modified phospholipid comprising a cell penetrating peptide - TAT - bonded onto the encapsulated core and entrapped by the PEG ligand;
wherein a remotely applied electromagnetic stimulus causes magnetic hyperthermia wherein the temperature-responsive PEG releases and exposes the phospholipid cell penetrating peptide to locally target cells and allow subsequent release of the active ingredient in to the target cells.

EP 4 205 768 A1

**Description**

**TECHNICAL FIELD**

The present disclosure relates to functionalized magnetic solid lipid nanoparticles. In particular, magnetic solid lipid nanoparticles comprising carnauba wax and a pharmaceutical composition for use in medicine. The present disclosure also relates to the functionalization of the nanoparticles' surface with two different functional ligands which is activated locally through magnetic hyperthermia.

**BACKGROUND**

Nanotechnology has become a promising strategy for controlled delivery of therapeutic agents. This strategy can compensate for the physical shortcomings of biologically-interesting molecules, and improve their overall properties. For example, many bioactive compounds that have been formulated in nanocarriers improved their solubility in water, physical and biological stability, biodistribution and other pharmacokinetic properties, in addition to their pharmacodynamics. Among the different types of nano-formulations capable of granting to some extent these effects to the cargo, solid lipid nanoparticles (SLNs) have shown particularly interesting characteristics. With optimal cargo characteristics, these biocompatible formulations can help avoid degradation and metabolic inactivation until the cargo's gradual liberation into the biological media, preventing side effects in a greater proportion. The system's high loading capacity also allows for the simultaneous loading of other components, including other bioactive compounds, dyes and inorganic effectors. For example, magnetic SLNs (mSLNs) encapsulate in their lipid core, in addition to the therapeutic cargo, magnetic nanoparticles (MNPs) that provide the nanosystem with their own physicochemical properties (i.e., magnetic properties). These properties have already been used to allow external interaction with the formulation via externally applied magnetic fields, allowing an additional level of control over the therapeutic-cargo release, enabling combinatorial therapy strategies (i.e., thermochemotherapy) and therefore improving the total therapeutic index in preclinical in vitro and in vivo validation models. Additionally, mSLNs displayed contrast-enhancing capabilities for magnetic resonance imaging (MRI) and subsequently have been tested and confirmed as effective diagnostic tools.

The general bioactivity of SLNs can be further enhanced by attaching and exposing a polyethylene glycol (PEG) polymer to the surface of the SLNs. The incorporation of PEG usually confers protection from the immune system, increases circulation times and reduces their uptake by the reticuloendothelial system. It also helps accumulate more in the tumour areas and less in the liver. Additionally, it is compatible with ligands that add a recognition element to the system. Amongst these ligands, small peptides have been widely explored. These include cell penetrating peptides (CPPs) that can be readily synthesized, are biocompatible and can be easily modified to fulfil overall strategical targeting and localized drug delivery purposes. CPPs possess the ability to cross cellular membranes themselves, and can facilitate the translocation of the attached and otherwise impermeable cargo and/or nanoparticles (NPs). They achieve this through their specific composition, where 10 to 30 mainly basic amino acids usually arrange amphipathically. It has been suggested that cationic CPPs can translocate through the membrane in an energy and receptor-independent way, most likely through an endocytic pathway. One of the first CPP discovered was the TAT peptide that was derived from the C-terminal of the HIV-TAT protein. Since its discovery, TAT has been extensively used to increase the translocation power of drugs and NPs alike.

Scientific publications on carnauba-wax carriers including for the delivering Doxorubicin (DOX) have also been made.

Several articles regarding the synthesis and applications of carnauba-wax-based mSLNs have been published.

The combination of a thermo-sensitive PEG nanoparticles with a Diels-Alder bond has been previously reported. Similarly, the use of a cell penetrating peptide in nanoparticles has also been reported.

Current formulations available in the market use chemical or biological moieties on the nanoparticle surface to selectively target and interact with the cells/tissues of interest. Although these strategies work relatively well in vitro, the translation to in vivo is highly hampered by limited to null targeting efficiency.

These facts are disclosed in order to illustrate the technical problem addressed by the present disclosure.

**GENERAL DESCRIPTION**

The present disclosure relates to functionalized magnetic solid lipid nanoparticles. In particular, magnetic solid lipid nanoparticles comprising carnauba-wax and a pharmaceutical composition for use in medicine.

The strategy of the present disclosure is based on the functionalization of wax nanoparticles' surface with two different functional ligands which is activated locally through magnetic hyperthermia.

Some advantages of the nanoparticles of the present disclosure are that they would allow pharmaceutical companies to (i) selectively deliver a drug to a specific target (i.e., chemotherapy to a tumour site), (ii) use low doses schemes, (iii) avoid systemic secondary effects and (iii) preserve drug stability.

The nanoparticle of the present disclosure also has the advantage that the targeting mechanism is triggered by the remote and local application of an electromagnetic stimulus (alternating magnetic field - AMF) in the target site. This avoids using membrane receptors on the cells' surface as targets, since the intracellular uptake of the carriers is driven by the exposure of the cell penetrating peptides (TATs) attached to the nanoparticle surface, which are exposed to the cells once the PEGylated ligands break down through their temperature-sensitive bond due to the temperature increase induced by the application of the AMF (magnetic hyperthermia). Therefore, the subject matter of this disclosure solves the problem of a lack of selectivity for delivering chemotherapeutics to those cell types without specific receptors. As an example, the nanoparticles of the present disclosure are advantageous in the context of triple negative breast cancer, a tumour cell type that tests negative for oestrogen receptors, progesterone receptors, and excess HER2 protein.

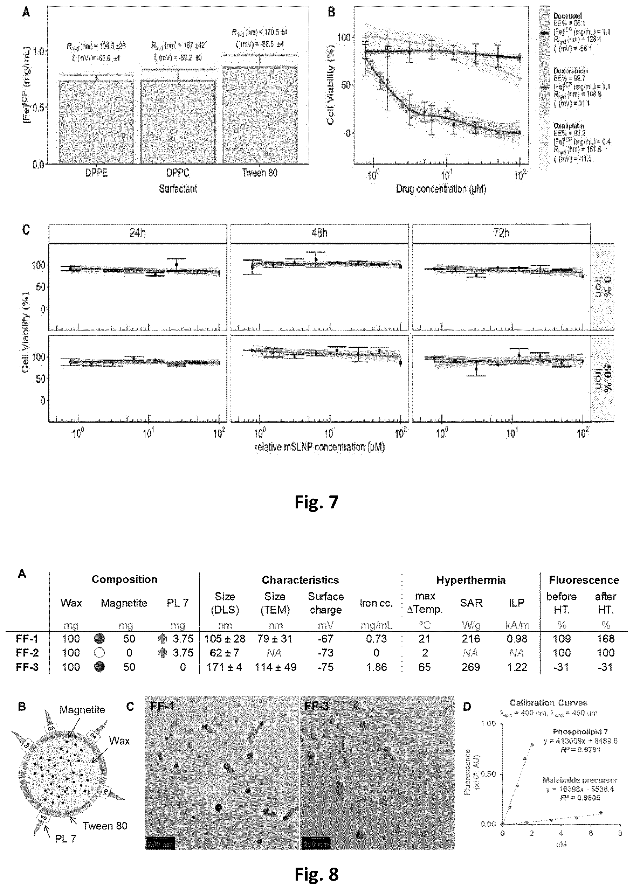

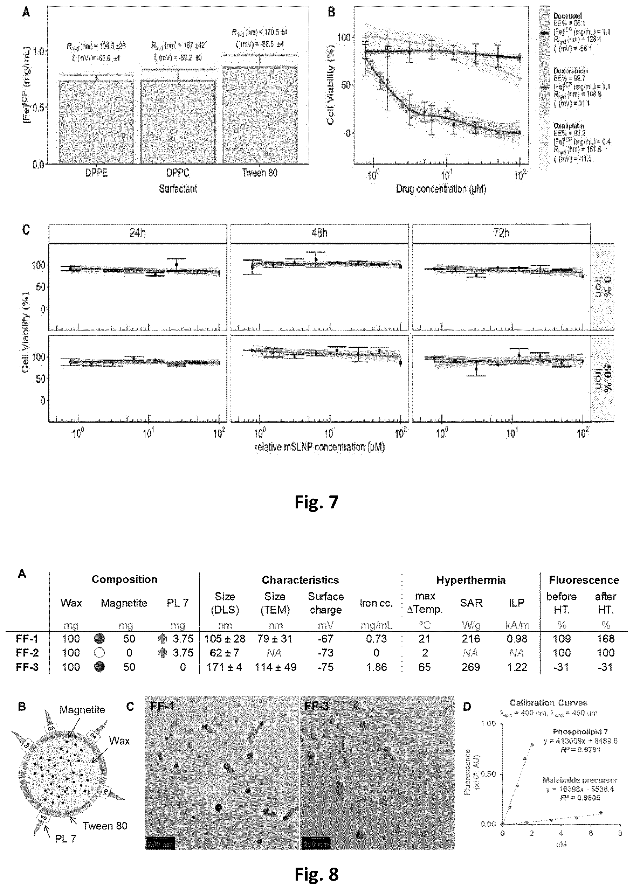

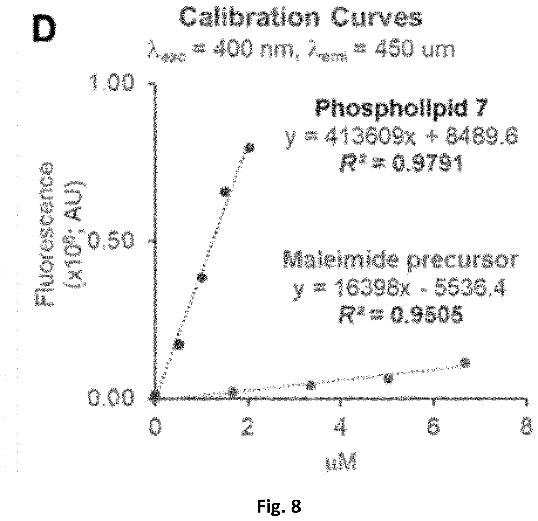

An aspect of the present disclosure relates to family of hybrid delivery magnetic solid lipid nanoparticles that are functionalized on their surface with a thermo-sensitive PEG ligand and a TAT ligand (the CPP), and loaded with chemotherapeutics, dyes and magnetite nanoparticles. The PEG ligand grants its bioactivity to the formulation whilst also protecting, shielding and sterically hindering the CPP. Upon application of an alternating magnetic field specifically to the tumour area, the locally concentrated magnetite inside the nanocarriers activates and irradiates heat through magnetic hyperthermia (MH) effect, which breaks-off the thermo-sensitive PEG ligand from the magnetic solid lipid nanoparticles (**Figure 8**). Such event will ultimately expose the CPP to the biological media and promote the nanoparticle's translocation to nearby cancer cells as means to gain selectivity. MH also allows for an extra level of control over the chemotherapeutic release process as well as potentially contributing with its own thermo-cytotoxicity. As thermo-sensitive bond that would allow the detachment of PEG, the retro Diels-Alder reaction presents very interesting characteristics. It is promoted by temperature and has been studied amply in the literature. Similarly, CPPs have been extensively studied and linked to drugs and formulations using versatile methodologies such as the Thiol-Michael addition click reaction, where cysteine is previously incorporated in the CPP chemical structure in order to form the stable C-S bond. Thus, the resulting responsive magnetic solid lipid nanoparticle has the potential to improve the bioactivity and therapeutic index of the chemotherapeutic cargo because of their controlled and selective release triggered by an external stimulus (local application of AMF-based MH).

An aspect of the present disclosure relates to the development of a remotely activatable magnetic solid lipid nanoparticle to target body tissues which are currently selectively not targetable by other means; the method of the present disclosure comprises the use of two magnetically-responsive phospholipid ligands to functionalize a lipid nanoparticle and locally deliver a cargo (active compound).

The targeting strategy described in the present disclosure was demonstrated through the functionalization of a developed magnetic wax vehicle used to transport lipophilic active compounds such as chemotherapeutics, dyes, and other inorganic compounds (i.e., magnetic nanoparticles) to a solid tumor in the context of triple-negative-breast-cancer, a breast cancer subtype which does not express either hormonal receptors or excess of HER protein. The whole system behaves as a theranostic platform that integrates diagnostic and therapeutic functionalities, and it can also work with new drugs or already approved drugs that need to improve their pharmaco-kinetic and -dynamic properties. This functionalization strategy could presumably be extended to other lipid-based carriers.

An aspect of the present disclosure relates to the synthesis of two different phospholipid-based molecules as responsive functional ligands for functionalizing magnetic solid lipid nanoparticles (mSLNs; the formulations). The core formulation comprises carnauba wax, iron oxide nanoparticles and, the surfactant Tween 80 which stabilizes the nanoparticles in water solution. In the present disclosure, the magnetic solid lipid nanoparticles are synthesized and stabilized in water solution using an additional mixture comprising two different molecules (ligands) as surfactants.

In an embodiment, one of the two surfactants comprises a phospholipid modified with a cell penetrating peptide ("TAT") that is known to help the translocation of the system attached to it to an inner cellular space.

In an embodiment, the second surfactant comprises a phospholipid modified with a poly-ethylene glycol ("PEG") moiety. When TAT ligand and PEG ligand are located together on the surface of the wax nanoparticle, the PEGylated ligand shields the TAT moiety with its long polymeric chain thus inactivating the function of the TAT ligand. Additionally, the PEGylated ligand is known to reduce the interaction with the immune system, thus prolonging the in vitro and in vivo stability of the formulation. Importantly, in the PEGylated ligand the PEG was coupled to the phospholipid backbone through a Diels-Alder bond which gives place to a chemical equilibrium that is sensitive to relatively high temperatures. Therefore, the continuous exposure of this PEG ligand to enough heat breaks the Diels-Alder bond through sequential electronic movement and provides the ligand with thermal-responsive properties. This trigger effect benefits from the incorporation of magnetic nanoparticles (iron oxide nanoparticles) into the wax cores (simultaneously together with a bioactive compound), which act as thermal effectors when an alternating magnetic field (AMF) is applied, converting the supplied electromagnetic energy into heat. This heat generates a temperature increase that forces the Diels-Alder bond to break and ultimately exposes the CPP-ligands. Once this happens, the drug-loaded nanoparticle can easily translocate into nearby cells and tissues and selectively deliver its cargo due to the intrinsic properties of the exposed TAT. Thus, the nanoparticles of the present disclosure can be used as a target-less strategy which can be precisely and selectively activated by the spatiotemporal application of an alternating magnetic field (AMF-hyperthermia). This is especially important to target cell types - such as triple negative breast cancer cells - that lack specific receptors on their membrane, since this strategy does not require cells expressing specific receptors for its targeting (which is the basis of conventional biological targeting strategies that show limited to null translation in vivo).

An aspect of the present disclosure relates to a magnetic solid lipid particle comprising:

an encapsulated core, wherein said core comprises hyperthermic sensitive magnetic nanoparticles, a surfactant, and an active ingredient;
a phospholipid modified poly-ethylene glycol molecule - PEG - bonded onto the surface of the encapsulated core and; wherein said PEG modified phospholipid is temperature sensitive;
a phospholipid cell penetrating peptide - TAT - entrapped by the PEG and bonded onto the encapsulated core;
wherein the surfactant stabilized the magnetic solid lipid particle;
wherein an electromagnetic stimulus on the lipid particle causes a release of the PEG in order to expose the phospholipid cell penetrating peptide to target cells and allow subsequent release of the active ingredient in to the target cell.

An aspect of the present disclosure relates to a magnetic solid lipid particle comprising:

an encapsulated core, wherein said core comprises thermal effectors and/or MRI contrast enhancers, a surfactant for stabilizing the magnetic solid lipid particle in aqueous solution, and an active ingredient;
wherein the thermal effectors and/or MRI contrast enhancers are magnetic nanoparticles;
a first modified phospholipid comprising a poly-ethylene glycol molecule - PEG - bonded onto the surface of the encapsulated core and; wherein said PEG modified phospholipid is temperature responsive; and
a second modified phospholipid comprising a cell penetrating peptide - TAT - bonded onto the encapsulated core and entrapped by the PEG ligand;
wherein a remotely applied electromagnetic stimulus causes magnetic hyperthermia wherein the temperature-responsive PEG releases and exposes the phospholipid cell penetrating peptide to locally target cells and allow subsequent release of the active ingredient in to the target cells.

In an embodiment, the surfactant is Tween 80, polysorbate 80, sorbitol, or mixtures thereof.

In an embodiment, the modified phospholipid is selected from phospholipid T-sensitive PEG, phospholipid cell penetrating peptide, or mixtures thereof.

In an embodiment, the core is a wax; preferably carnauba wax, bee wax, or mixtures thereof.

In an embodiment, the thermal effectors magnetic nanoparticles are magnetite nanoparticles, iron oxide nanoparticles, or mixtures thereof.

In an embodiment, the MRI contrast enhancers magnetic nanoparticles are magnetite nanoparticles, iron oxide nanoparticles, or mixtures thereof.

In an embodiment, the PEG molecular weight ranges from 2000 Da to 10000 Da, preferably 5000 Da.

In an embodiment, the active ingredient is an anticancer drug, an antimalaria drug, an antifungal drug, an antibiotic, an immune-modulator, a calcium blocker, or a vitamin.

In an embodiment, the anticancer drug is docetaxel, oxaliplatin, doxorubicin, 5-Fluorouracil, Cisplatin, Bortezomib, or mixtures thereof.

In an embodiment, the antimalaria drug is artemisinin.

In an embodiment, the immune-modulator is imiquimod, resiquimod, or mixtures thereof.

In an embodiment, the antifungal drug is terbinafine, tavaborole.

In an embodiment, the antibiotic is Erythromycin, Azithromycin, Chloramphenicol, Enrofloxacin.

In an embodiment, the vitamin is biotin.

An aspect of the present disclosure relates to a pharmaceutical composition comprising the lipid particle of the present disclosure, for use in medicine or as a medicament.

In an embodiment, the pharmaceutical composition is for use as a chemotherapy agent, as a thermotherapy agent, as thermochematherapeutic agent and as a theranostic agent.

In an embodiment, the pharmaceutical composition is for use in the treatment of a solid cancer.

In an embodiment, the pharmaceutical composition is for use in the treatment of breast cancer.

In an embodiment, the pharmaceutical composition is for use in the treatment of triple negative breast cancer.

An aspect of the present disclosure relates to a method of preparing the lipid particle of the present disclosure comprising:

melting wax;

adding surfactant, modified phospholipid and active ingredient to the melted wax to form a suspension;
subjecting the suspension to ultrasonication;
placing the ultrasonicated suspension in a cold bath to solidifying lipid nanoparticles in the suspension;
centrifugating the suspension to obtain supernatant comprising the lipid nanoparticles.

## BRIEF DESCRIPTION OF THE DRAWINGS

The following figures provide preferred embodiments for illustrating the disclosure and should not be seen as limiting the scope of invention.

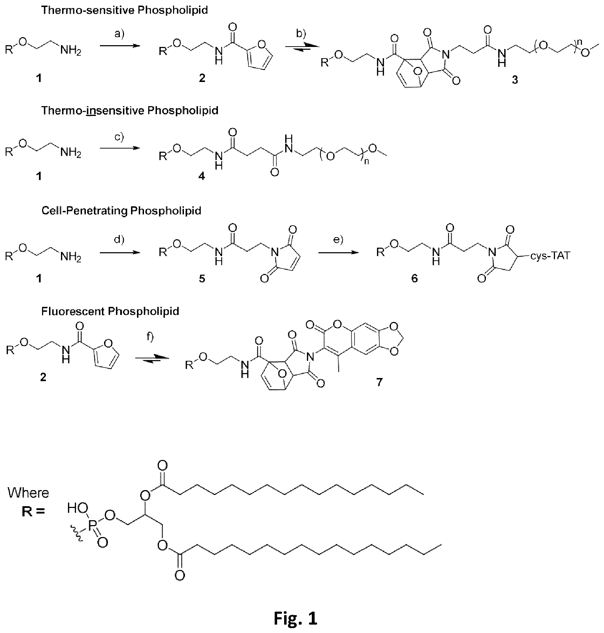

**Figure 1** shows the chemical structure of both control and functional ligands.

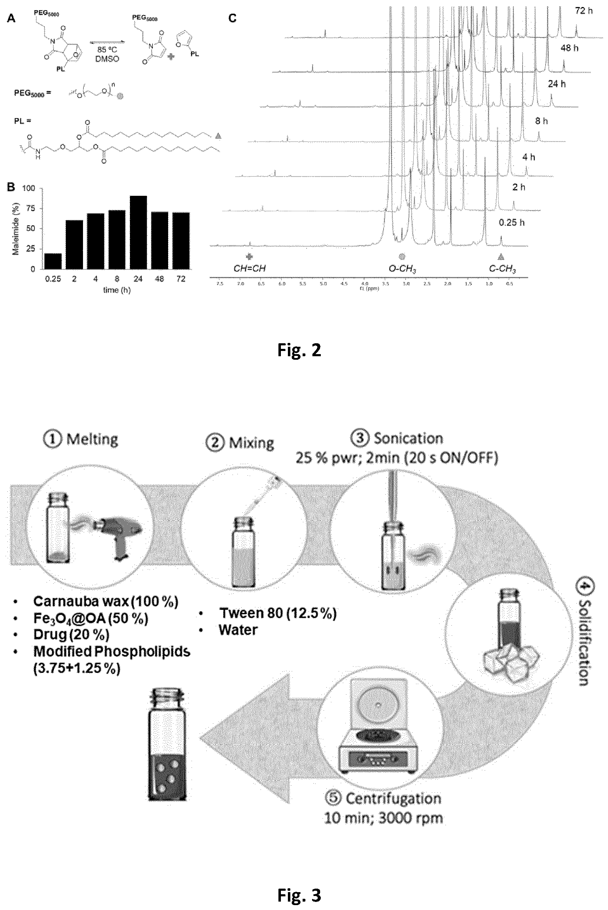

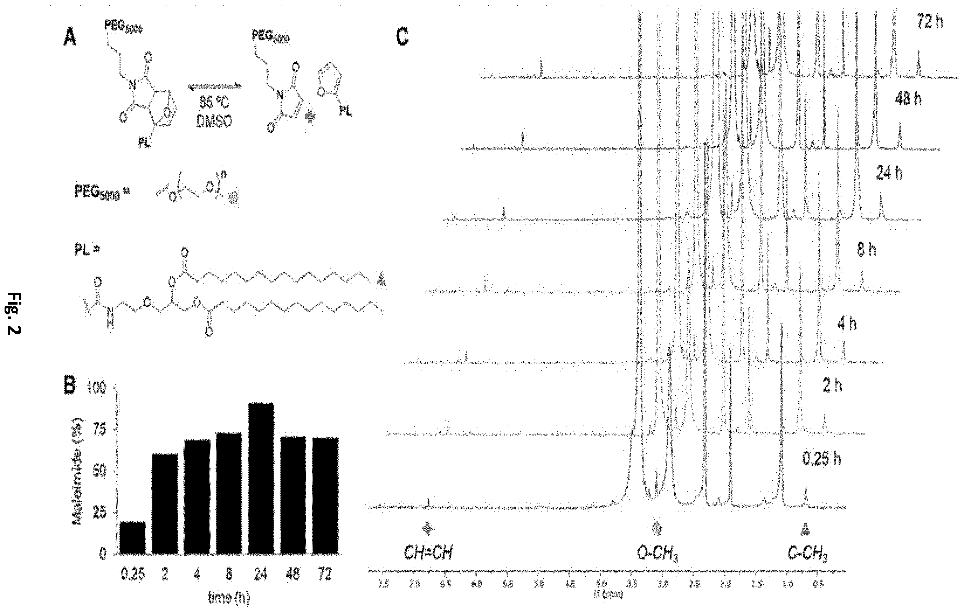

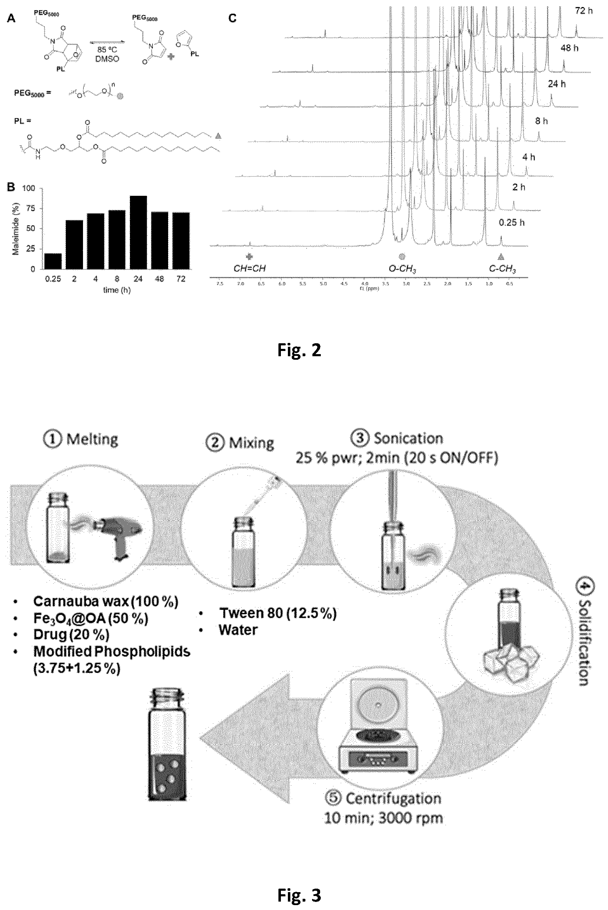

**Figure 2A** shows the Diels-Alder equilibrium (incorporated in the PEGylated ligand to provide it with thermo-responsive properties). The yellow circle and grey triangle are the methyl groups of the PEG and fatty acids, respectively, used to quantify the abundance of the maleimide protons (blue cross). **Figure 2B** shows the time evolution of the equilibrium using the signal of the maleimide moiety as probe. 100 % of the maleimide moiety indicates the complete dissociation of the equilibrium whilst 0 % indicates the transformation to the Diels-Alder adduct. **Figure 2C** shows time-resolved NMR spectroscopy.

**Figure 3** shows the melt-emulsification synthetic protocol used to prepare the functionalized magnetic solid lipid nanoparticles.

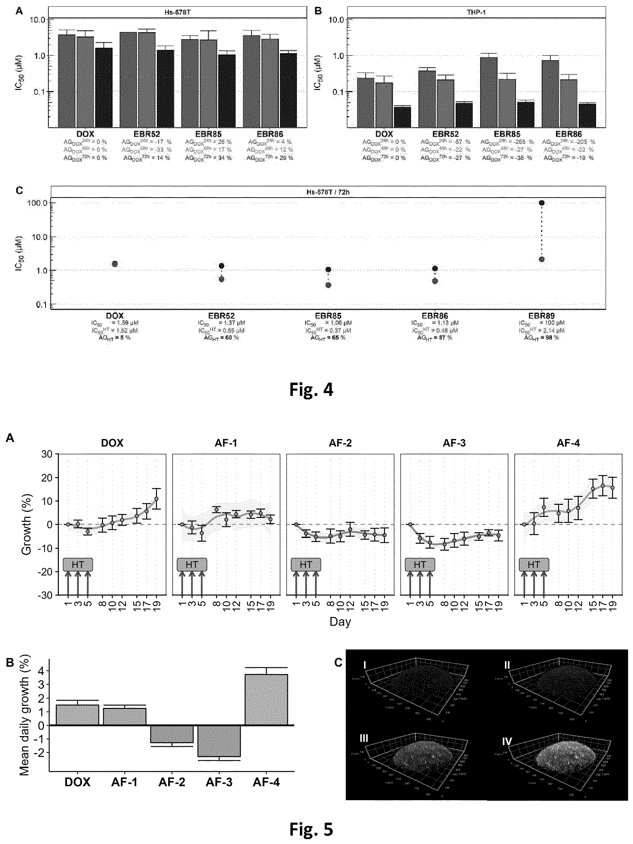

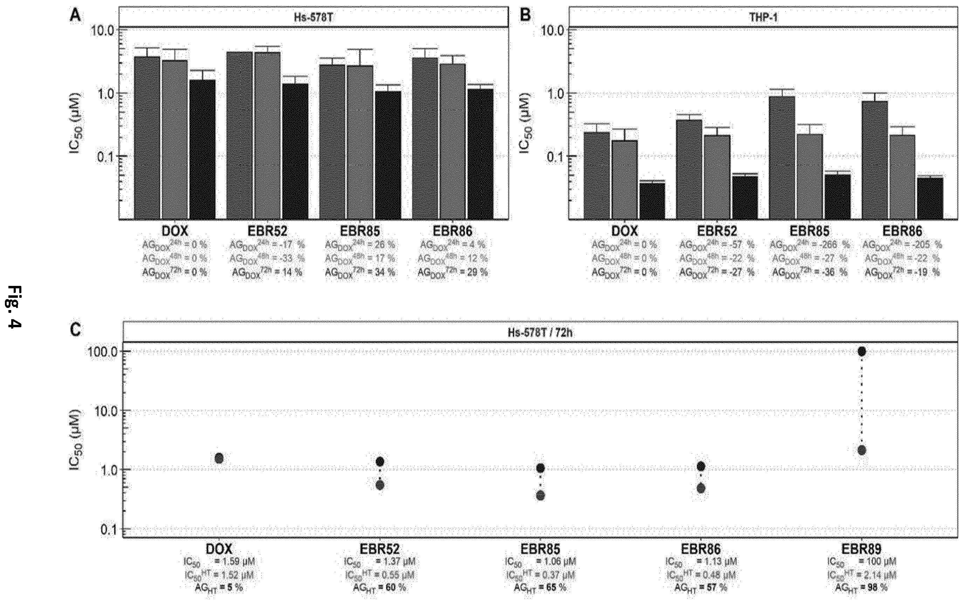

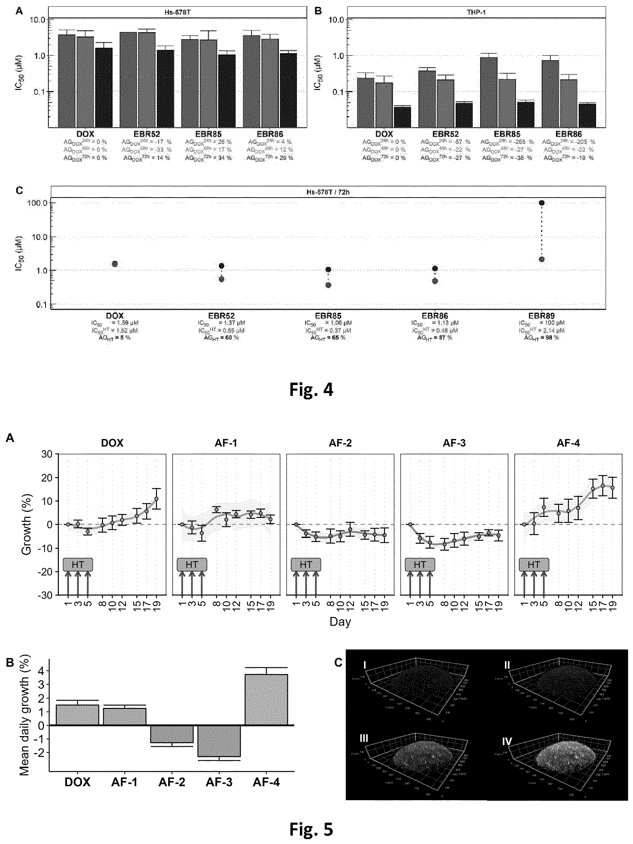

**Figure 4A** shows the cell viability of triple negative breast cancer cells (Hs-578T) at inhibitory concentration 50 (IC50) for 3 different times (24 h- purple; 48 h- dark purple; 72 h- blue). The activity gain compared to the free DOX is given underneath as a percentage. Positive gain means an improvement; whilst negative gain indicates a reduction of activity compared to free DOX. **Figure 4B** shows the cell viability of THP-1 macrophages which are known to avoid interaction with the PEG moiety. Figure 4C shows the cell viability of triple negative breast cancer cells (Hs-578T) at inhibitory concentration 50 (IC50) with and without 1h of magnetic hyperthermia (red/blue, respectively) after additional 72 h of incubation. The activity gain of the hyperthermia event is given for each formulation.

**Figure 5** shows A) the time evolution of Hs-578T polyspheroids growth treated with the different active formulations. Their growth was monitored three weeks, the first of which (in days 1, 3 and 5) we applied the dual treatment comprising incubations with the mSLNs (2 μM, relative to DOX concentration within the mSLNs) followed by their exposure to an AMF for 1h. Four polyspheroids were used per condition in two independent experiments. Each polyspheroids' initial volume (day 1) was used as individual reference from which its posterior growth was calculated; B) the mean daily polyspheroids growth per condition (as the sum of the total growth/19 days); and C) confocal images of the top of a polyspheroid treated with Hoescht dye (I, blue), DiD within AF-2 (II, red), DiO (III, green) and the merge of all channels (IV). AF-2 corresponds to the temperature-sensitive mSLNs containing DOX.

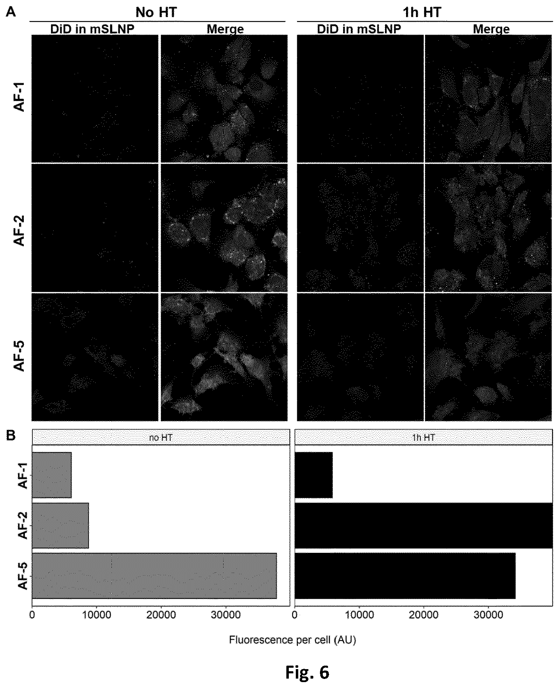

**Figure 6A** shows confocal microscopy images of the DiD-loaded formulations with phospholipids 3 and 6 (first row), phospholipid 6 (second row) and without phospholipids (third row). The first and second column are the images obtained in the DiD channel and the merge of all channels, respectively, for 3h incubation without magnetic hyperthermia treatment. The third and fourth columns are the DiD channel and the merge of all channels, respectively, after 1h magnetic hyperthermia treatment and an additional 2h of incubation. Figure 6B shows the cellular fluorescence quantification under the different conditions, without and with magnetic hyperthermia application.

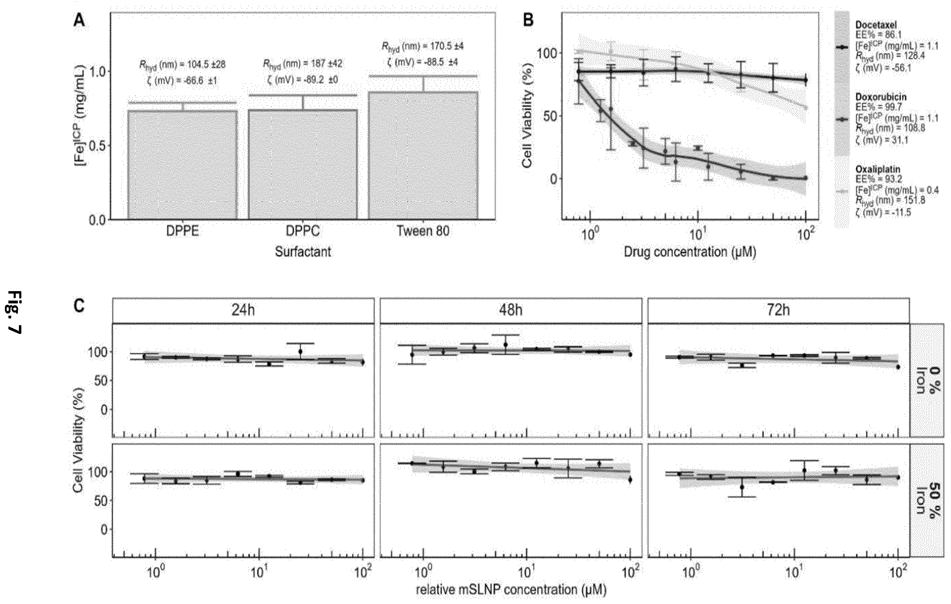

**Figure 7** shows results of the optimization cycles (Figure 7A) when optimizing the iron loading, size and surface charge using different surfactants and (Figure 7B) when employing different active compounds loaded in the nanoparticles. Their physical characteristics, drug encapsulation efficiency (EE%) and bioactivity (in a Hs-578T cell viability model after 72 h of incubation) are displayed. Figure 7C shows the cytotoxic activity of the drug-unloaded formulations (as to determine the effect of the wax, magnetite, and surfactants) evaluated in the same viability model whilst using equal concentrations relative to their drug-loaded counterparts (in concentration of DOX, μM).

**Figure 8A** shows control fluorescent formulations (FF) prepared (with an additional 12.5 mg of Tween 80 in 2.25 mL of water) and characterized to verify the incorporation of the phospholipids around the formulations. These mSLNs were also studied to confirm the Diels-Alder equilibrium shift in water, and the eventual heat-activated fluorescent break-off caused by an alternating magnetic field of 896 MHz and 200G. Concentration is abbreviated cc., phospholipids are abbreviated PL, hyperthermia is abbreviated HT, maximum change in temperature is abbre-

viated max ∆Temp. Figure 8B is a conceptualization image of the mSLN prepared. Figure 8C shows TEM images of the mSLNs formulations. **Figure 8D** shows a fluorescence intensity comparison and calibration curves for phospholipid 7's Diels-Alder equilibrium species. The coumarin-conjugated phospholipid (DA adduct) is 25x fold more fluorescence than the same concentration of the maleimide-coumarin precursor (DA reagents).

**Figure 9** shows physicochemical characterization results of active formulations (AF). **Figure 9A** shows the conceptualization scheme of the thermo-responsive mSLN prepared and targeting mechanism; **Figure 9B** shows TEM images of the magnetite loaded formulations; **Figure 9C** shows the compositional and physicochemical characterization results, including doxorubicin encapsulation efficiency (DOX. EE.) as well as magnetic hyperthermia and relaxivity efficiency.

**Figure 10** shows AF-1 and AF-2 sixty hours passive release profiles of DOX (top row). The % DOX released vs time data best fitted a sigmoidal/logarithmic model (lowest AIC). The DOX release profiles without and with application of an alternating magnetic field (896 kHz, 200 G, applied during the first hour of release) were compared during the initial 4 h. A linear model fitted well the first hours of DOX release.

**Figure 11** shows AF-1, AF-2 and AF-3 TEM images before and after the application of an AMF for 1h (896 kHz, 200 G) at different magnifications.

**Figure 12** shows the active formulations T2 relaxation times per iron concentration in aqueous solution.

**Figure 13** shows the T2 relaxation times per active formulation tested of Hs578T polyspheroids. MRI was done in day 19 of the TNBC polyspheroid growth study. Images were taken on eppendorfs and in compartmentalized petri dishes. * denotes a significance of at least $p < 0.05$.

**Figure 14** shows optical images to highlight the effect of mSLNs on migration capabilities of HS-578T cells after 0, 6, 24, 36 and 48h of incubation with mSLNs (with and without DOX) at the $IC_{50}$ DOX concentration and equivalent mSLNs, respectively. AF-2 corresponds to the T-sensitive mSLNs, whereas AF-3 corresponds to the T-insensitive mSLNs as control.

**Figure 15** shows the graphical representation of wound closure percentage (cell migration) relative to control group (DMEM medium). Results are presented as mean ± SD of at least three independent experiments.

**Figure 16** shows the effect of the T-sensitive and DOX-loaded mSLNs (AF-2 = EBR85) nanoparticles and free DOX on Hs578t tumor growth in an *in ovo* model. (A) *In ovo* pictures at day 13 of embryo development and *in ovo* and *ex ovo* pictures at day 17; representative images were taken at 20x in a Zeiss stereomicroscope. (B) Graphical representation of tumor growth. Data was analyzed by one-way ANOVA and the results are representative for the mean of at least 15 eggs per group;***p<0.0001 *p<0.05.

**Figure 17** shows a visual concept note highlighting the drug-loaded and functionalized mSLN and its application to treat triple-negative-breast-cancer.

## DETAILED DESCRIPTION

The present disclosure relates to functionalized magnetic solid lipid nanoparticles. In particular, magnetic solid lipid nanoparticles comprising carnauba-wax and a pharmaceutical composition for use in medicine.

The present disclosure also relates to the functionalization of the magnetic solid lipid nanoparticles' surface with two different functional ligands which is activated locally through magnetic hyperthermia.

In an embodiment, the nanoparticle of the present disclosure comprises two different ligands, one of them being pH-sheddable and the other containing a CPP moiety. To demonstrate the efficiency of this targeting strategy, both control and functional ligands were synthesized using organic chemistry protocols through the following synthetic routes: As illustrated in **Figure 1**, compound 3 is the thermo-sensitive Diels-Alder PEGylated ligand, compound 4 is its thermo-resistant control, compound 6 is the functionalized ligand with the cell penetrating peptide (TAT) and compound 7 is a fluorescent ligand.

In an embodiment, as illustrated in **Figure 1**, the reagents and conditions are as follows: (a) PyBOP, DIPEA, $CHCl_3$ anh., 24 h. (b) α-Methoxy-Ω-ethyl-maleimidepoly(ethylene glycol)$_{5000}$, Toulene, 90 °C. (c) α-Methoxy-Ω-carboxylic acid succinimidyl ester poly(ethylene glycol)$_{5000}$, TEA, $CHCl_3$ anh. 4 h. (e) cyc-TAT, 12 h. f) 6, 7-Methylenedioxy-4-methyl-3-maleimidocoumarin, Toulene, 90 °C.

In an embodiment, the modified phospholipids were characterized by Nuclear Magnetic Resonance (NMR) and/or High Resolution Mass Spectroscopy (HRMS). For Phospholipid 3 (and 7), the Diels-Alder reaction product was found to be a mixture of endo and exo products. Hence, to confirm the formation of the final product by NMR, the maleimide signal (at 6.7 ppm) was compared to signals that are not expected to change throughout the evolution of the Diels-Alder equilibrium. Its disappearance confirmed the formation of the Diels-Alder product. Thus, NMR analysis of the evolution of the Diels-Alder equilibrium was performed with phospholipid 3 for 72h in DMSO. The signal characteristic of the dissociated state of the equilibrium (blue cross in **Figure 2A**), was quantified and compared with signals that are expected not to be affected overtime (yellow circle and grey triangle, **Figure 2A**). The increase of the signal indicates the fast conversion of the Diels-Alder product to its constituent parts after applying temperature in DMSO. (See **Figure 2B** and C) **Figure 2A** shows the Diels-Alder equilibrium. The yellow circle and grey triangle are the methyl groups of the PEG and fatty acids, respectively, used to quantify the abundance of the maleimide protons (blue cross). **Figure 2B** shows time evolution of the equilibrium using the signal of the maleimide moiety as probe. 100 % of the maleimide moiety represents the complete dissociation of the equilibrium whilst 0 % indicates the transformation to the Diels-Alder adduct. **Figure 2C** shows time-resolved NMR spectroscopy.

In an embodiment, several new doxorubicin-loaded magnetic nanoparticles formulations were prepared using a modified melt emulsification method. Initially, 100 mg of Carnauba wax were mixed in a glass vial with 50 mg of magnetite in chloroform (0.264 mL of a solution 189 mg/mL) and the drug Doxorubicin (20mg). Then the functionalized phospholipids (3.75 % of compound 3, 4 or 7 and/or 1.25 % of compound 6; 5 % in total) were added. The chloroform was then evaporated and thereafter 1.75 mL of water was added. The resulting suspension was heated. 12.5 % of Tween 80 (0.5 mL of a solution 25 mg/mL) were then added to the vial (2.25 mL total water). The sample was ultrasonicated for 2 min at 25% power at 20 second working intervals while being simultaneously heated with a heat gun. Immediately after the sonication, the sample was immersed in ice to solidify the lipid nanoparticles. Once cold, a 300 μL aliquot was taken from the vial to later quantify the encapsulation efficiency. Then, the formulation was centrifuged to remove aggregates (3000 RPM, 10 min), the supernatant was stored in a glass vial and the pellet was discarded. Protocol described in **Figure 3.**

In an embodiment, the nanoparticles were able to encapsulate the drug (Doxorubicin) efficiently (EE% > 97 %), and iron in concentrations over 1 mg/mL in the stock solution.

In order to confirm the efficiency of the magnetic hyperthermia-induced PEG-cleavage mechanism in the final magnetic solid lipid nanoparticles, the fluorescence change of the equilibrium was studied with nanoparticles synthesized with 3.75 % of phospholipid 7, where the fluorescence probe is bonded to the rest of the phospholipid through a Diels-Alder link. After an alternating magnetic field application of 1 hour, the fluorescence intensity due to the dissociated maleimide-fluorophore increased by 81 %, whilst the formulations without magnetite and/or without the modified surfactant 7 did not induce an increase in fluorescence after the same hyperthermia conditions. This confirms the evolution of the retro Diels-Alder also in water, since the NMR analysis described in [0047] confirmed it in DMSO.

**Table 1** below shows the formulations used for fluorescence spectroscopy. ΔTemp is the change of temperature observed because of the application of an alternating magnetic field (896 kHz, 200 G) for 1h.

| Formulation | | | Relative Fluorescence (%) | |
| --- | --- | --- | --- | --- |
| Magnetite (50%) | Modified Surfactant 7 (3.75%) | Δ Temp | Before heat treatment | After heat treatment |
| Yes | Yes | 23 | 119 | 200 |
| No | Yes | 2 | 100 | 97 |
| Yes | No | 67 | -31 | -31 |

In an embodiment, in order to demonstrate the in vitro efficacy of the nanoparticles of the present disclosure, the cytotoxicity effect of the DOX-loaded magnetic solid lipid nanoparticles functionalized with the mixture of functional ligands - the PEG-sheddable (Phospholipid 3) and the CPP (TAT)-containing (Phospholipid 6) surfactants - was assessed. The cytotoxic activity of the formulations was evaluated in a triple negative-breast-cancer cell line (Hs-578T) and macrophages (THP-1; **Figure 4A** and **Figure 4B** respectively).

Hs-578T cell line was used since these breast cancer cells do not express estrogen receptors (ER), progesterone receptors (PR) or amplification of HER-2/Neu, and therefore is extremely difficult to selectively target them via traditional biological and/or chemical strategies. These specific cancers represent approximately 10-15% of all breast cancers; patients with them have a poor prognosis as compared to other subtypes of breast cancer. As a potential solution, the strategy proposed here can greatly benefit the selective activity of chemotherapy because it does not require the presence of any specific receptor. Instead, its selectivity arises from the local application of a magnetic field near the tumour area that activates locally the internalization of the nanoparticles, drug release and over all its cytotoxic activity.

THP-1 cell line was also used because they are known to phagocyte nanoparticles and deactivate them, hence being a limiting factor for the system. However, the presence of the PEG moiety on the surface of the nanoparticles is also known to decrease their overall clearance compared to non-PEGylated nanoparticles, thus increasing the circulation time in the blood stream after systemic administration (in an in vivo scenario). Therefore, this cell line constitutes a suitable model to phenotypically prove the effect and presence of PEG on the magnetic solid lipid nanoparticle surface.

The cytotoxicity was initially studied in these two cell lines without magnetic hyperthermia activation to analyze the effect of the DOX-loaded and functionalized magnetic solid lipid nanoparticles as compared to that of free DOX. The results of the DOX-loaded magnetic solid lipid nanoparticle's activity relative to the administration of the same concentration of unformulated DOX were shown in **Figures 4A and 4B.** This information was then used to calculate the magnetic solid lipid nanoparticle's activity increase with and without magnetic hyperthermia as compared to that of control formulations (**Figure 4C**).

Several control and sample magnetic solid lipid nanoparticles functionalized with different ligands combinations, w/o magnetite and DOX, were used in the validation experiments:

- DOX: free DOX

- EBR52: mSLNs with DOX, magnetite (without functional ligands)

- EBR85: mSLNs with DOX, magnetite and functional ligands (phospholipids 3 & 6: PEG-sheddable and TAT ones)

- EBR86: mSLNs with DOX, magnetite and non-functional phospholipids 4 & 6: PEG-insensitive and TAT

- EBR89: mSLNs with magnetite and functional ligands (phospholipids 3 & 6: PEG-sheddable and TAT (without DOX)

On the one hand, all the mSLNs without DOX, independent of the functionalized phospholipids and whether or not they contain magnetite were observed to be nontoxic in the whole concentration range tested (> 100 $\mu$M).

On the other hand, for those DOX-loaded formulations, results showed that the magnetic solid lipid nanoparticles loaded with DOX and magnetite, and functionalized with the phospholipids 3 and 6 (EBR85) displayed the highest cytotoxic activity increase compared to that of the free DOX in triple negative breast cancer cells (Hs-578T) (by 34 % after 72h). Under magnetic hyperthermia, this formulation (EBR85) displayed an extra cytotoxic activity increase of 65 % (**Figure 4C**). Thus, the cytotoxicity activity increase relative to the same amount of unformulated DOX was 76 % ($IC50_{DOX}$=1.52 $\mu$M to $IC50_{EBR85}$=0.37 $\mu$M).

In the case of the macrophages, the presence of the PEG in EBR85 prevented its internalization and hence hindered the activity of DOX when incubated for 24 hour (-266 % activity increase). This preventive effect was however gradually lost with longer incubation time. **Figure 4A** shows the cell viability of triple negative breast cancer cells (Hs-578T) at inhibitory concentration 50 (IC50) for 3 different times (24 hours purple; 48 hours dark purple; 72 hours blue). The activity increase compared to the free DOX is given underneath as a percentage. Positive increase means an improvement; whilst negative increase indicates a reduction of activity compared to free DOX. **Figure 4B** shows the cell viability of THP-1 macrophages, which are known to avoid interaction with the PEG moiety.

In an embodiment, the hyperthermia-induced targeting mechanism was also confirmed in 3D models (polyspheroids) of triple negative-breast-cancer cells (Hs-578T) **(Figure 5A).** The functionalized formulation activated by magnetic hyperthermia (EBR85) outperformed both the free DOX and the formulated DOX without the functionalized phospholipids (EBR52). The formulation (EBR85) was able to reduce the cancerous tumor volume instead of just slowing down its growth, as the free drug and EBR52 did.

**Figure 5B** shows the normalized spheroid growth per time, after the administration of each condition and magnetic hyperthermia on day 1, 3 and 5.

Figure 5C shows confocal microscopy (CLSM) images where the internalization of the sample formulation (EBR85, labelled with the green fluorophore DiO) in the Hs-578T polyspheroids is confirmed, as well as the internalization of DOX (red fluorescence).

In an embodiment, internalization of each magnetic solid lipid nanoparticle in Hs-578T triple-negative-breast cancer polyspheroids was studied using confocal microscopy and nanoparticles labelled with the red fluorophore DiD, but without DOX. After 3 hours of incubation, the formulation functionalized with the phospholipids of interest barely managed to enter the cells (first row of **Figure 6**, columns 1 and 2), in a similar fashion to the control formulation without the functional phospholipids (third row, columns 1 and 2). However, when magnetic hyperthermia is applied for 1h and the cells were additionally incubated for 2h, the formulation with the functionalized phospholipids (first row, columns 3 and 4) was able to increase its internalization in a similar fashion to the formulation prepared only with the cell penetrating peptide (which is not hindered by the PEG moiety, row 2) (see **Figure 6B**). **Figure 6** shows confocal microscopy images of DiD-loaded formulations with phospholipids 3 and 6 (first row), phospholipid 6 (second row) and without phospholipids (third row). The first and second column are the images obtained in the DiD channel and the merge of all channels, respectively, for 3h incubation without magnetic hyperthermia treatment. The third and fourth columns are the DiD channel and the merge of all channels, respectively, after 1h magnetic hyperthermia treatment and additional 2 hours of incubation.

The magnetic solid lipid nanoparticle preparation of the present disclosure was first optimized exploring several factors during its synthesis.

Initially, the magnetic solid lipid nanoparticles were prepared using three different surfactants (DPPE, DPPC and Tween 80) in addition to the combination of both functional pegylated T-sensitive ligand and CPP-ligand). DLS and ICP were employed to characterize the physicochemical properties of the mSLNs, as well as the amount of iron (coming from magnetite nanoparticles) encapsulated, thus allowing to select the best suitable surfactant to prepare the formulations (see Figure 7A). Tween 80 was selected because it managed to encapsulate more magnetite than the others, whilst maintaining the desired hydrodynamic size of the formulation, in addition to be the "cheapest" and most described surfactant in literature (also FDA approved).

Thereafter, different active compounds were used to be encapsulated in the magnetic solid lipid nanoparticles, and after characterization by DLS, ICP and HPLC (to determine the encapsulation efficiency, as EE%), the nanoparticles were evaluated on TNBC 2D cultures (cell line Hs-578T) after 72 hours of incubation (see **Figure 7B**). Doxorubicin managed to get encapsulated more efficiently than Docetaxel and Oxaliplatin.

The cell viability results obtained for Hs-578T cells after incubation with increasing concentration of magnetic solid lipid nanoparticles (with 3.75 % of pegylated T-sensitive ligand, 1.25 % of the CPP-ligand and 12.5 % of Tween 80) loaded with different drugs can be observed in Figure 7B.

Magnetic solid lipid nanoparticles loaded with Doxorubicin were also much more active on the cell lines employed, whilst the other drugs were barely toxic at the maximum concentrations tested. These results were similar to those observed when the free drugs were tested, where no significant toxicity was appreciated at the maximum concentration tested (100 uM).

Also, it is noticeable that the formulation with Oxaliplatin significantly diminished the iron encapsulation in the nanoparticles, which is not appreciable when theother drugs are incorporated.

The activity of Docetaxel, Oxaliplatin and Doxorubicin are consistent with reports in literature at the concentrations tested, being DOX the one showing the lower IC50. To evaluate the other drug-loaded formulations (Docetaxel, oxilaplatin) higher concentrations should be used ideally in more sensitive cell lines (for example, normal MCF7 breast cancer cell line).

The in vitro activity of drug-free magnetic solid lipid nanoparticles (with 3.75 % of pegylated T-sensitive ligand, 1.25 % of the CPP-ligand and 12.5 % of Tween 80) was evaluated in 2D Hs-578T cells. The presence of iron, wax, Tween 80 and the functional ligands did not cause any toxic effect on the cells at any of the three time points at which the activity was evaluated. Hence, the activity of the formulations is exclusively dependent on the drug loaded and its release profile.

**Figure 7** shows results of the optimization cycles (**Figure 7A**) when optimizing the iron loading, size and surface charge using different surfactants and (**Figure 7B**) when employing different active compounds to load in the nanoparticles. Their physical characteristics, drug encapsulation efficiency (EE%) and bioactivity (in a Hs-578T cell viability model incubated for 72 h) were considered. Figure 7C shows the cytotoxic activity of the drug-unloaded formulations (as to determine the effect of the wax, magnetite, and surfactants) evaluated in the same viability model whilst using equal concentrations relative to their drug-loaded counterparts (in concentration of DOX, $\mu$M).

**Figure 8A** shows control fluorescent formulations (FF) prepared (with an additional 12.5 mg of Tween 80 in 2.25 mL of water) and characterized to verify the incorporation of the phospholipids around the formulations. These mSLNs were also studied to confirm the Diels-Alder equilibrium shift in water, and the eventual heat-activated fluorescent break-off caused by an alternating magnetic field of 896 MHz and 200G. Concentration is abbreviated cc., phospholipids is abbreviated PL, hyperthermia is abbreviated HT, maximum change in temperature is abbreviated max $\Delta$Temp. Figure 8B is a conceptualization image of the mSLNs prepared. Figure 8C shows TEM images of the mSLNs formulations. Figure 8D shows a fluorescence intensity comparison and calibration curves for phospholipid 7's Diels-Alder equilibrium species. The coumarin-conjugated phospholipid (DA adduct) is 25x fold more fluorescence than the same concentration of the maleimide-coumarin precursor (DA reagents).

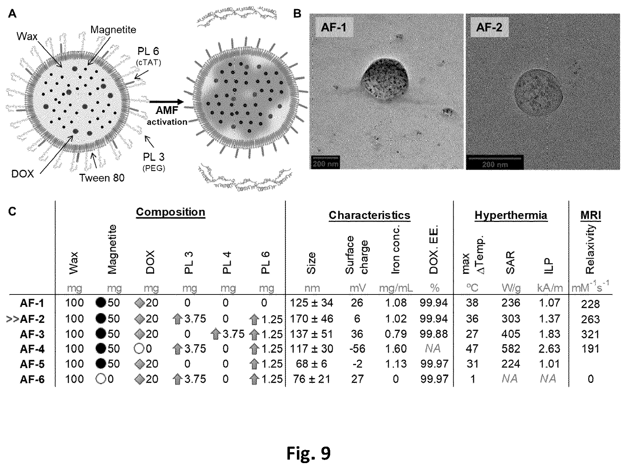

**Figure 9** shows physicochemical characterization results of active formulations (AF). **Figure 9A** shows the conceptualization scheme of the thermo-responsive mSLN prepared and targeting mechanism; **Figure 9B** shows TEM images of the magnetite loaded formulations; **Figure 9C** shows compositional and physicochemical characterization results, including doxorubicin encapsulation efficiency (DOX. EE.) as well as magnetic hyperthermia and relaxivity efficiency.

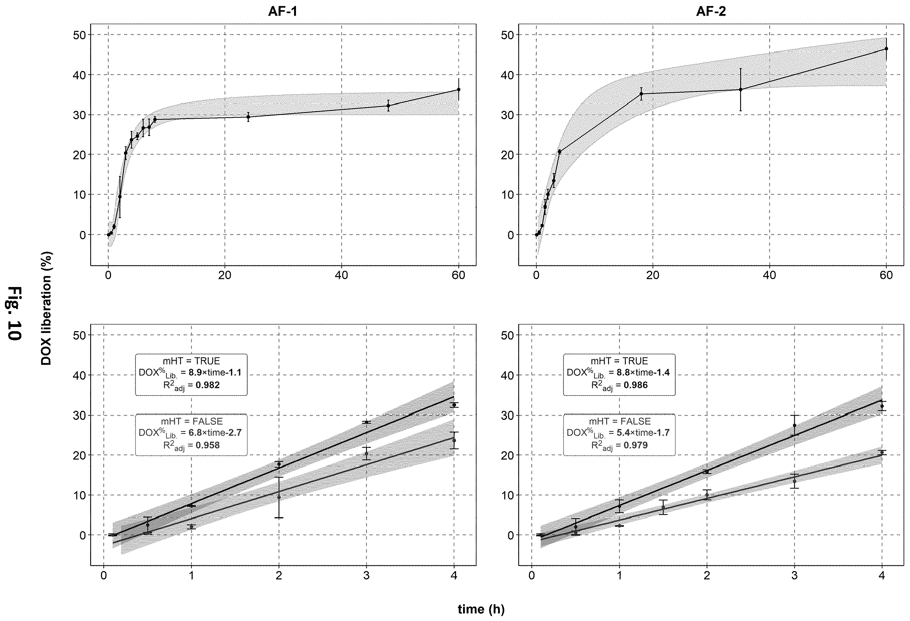

**Figure 10** shows AF-1 and AF-2 sixty hours passive release profiles of DOX (top row). The % DOX released vs time data best fitted a sigmoidal/logarithmic model (lowest AIC). The DOX release profiles without and with application of an alternating magnetic field (896 kHz, 200 G, applied during the first hour of release) were compared during the initial 4 h of release. A linear model fitted well the first hours of DOX release.

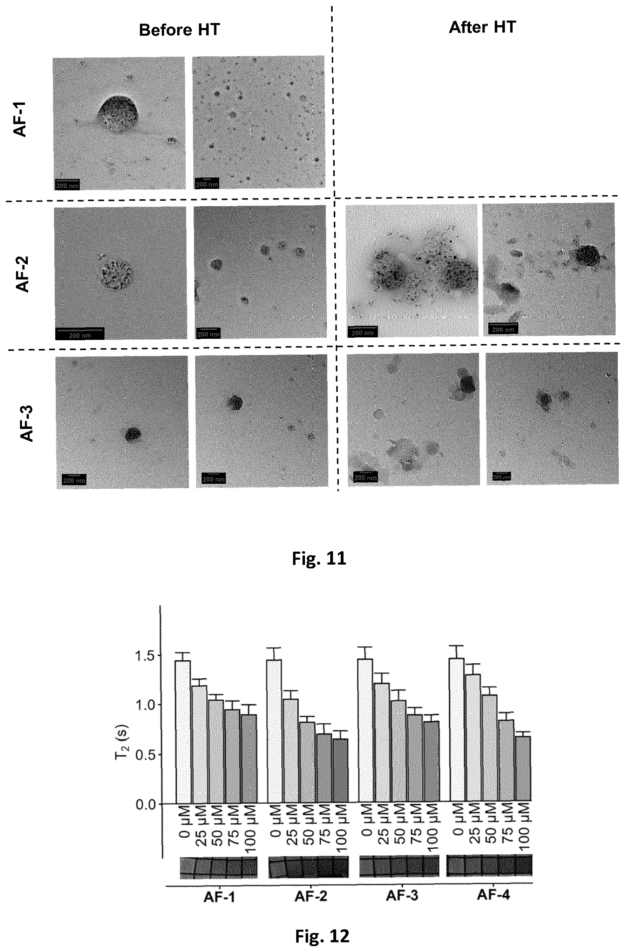

**Figure 11** shows AF-1, AF-2 and AF-3 TEM images before and after the application of an AMF for 1h (896 kHz, 200 G) at different magnifications. Results clearly show structural and morphological damages in the mSLNs after heat treatment through magnetic hyperthermia.

**Figure 12** shows the active formulations T2 relaxation times per iron concentration in aqueous solution. A concentration-dependent darker effect is observed as the concentration of mSLNs increases (higher iron concentration in solution), which is translated into a reduction of the transversal relaxation time (T2). This confirms the mSLNs as efficient T2-MRI contrast agents, thus becoming theranostic agents able to integrate diagnostic and therapeutic properties.

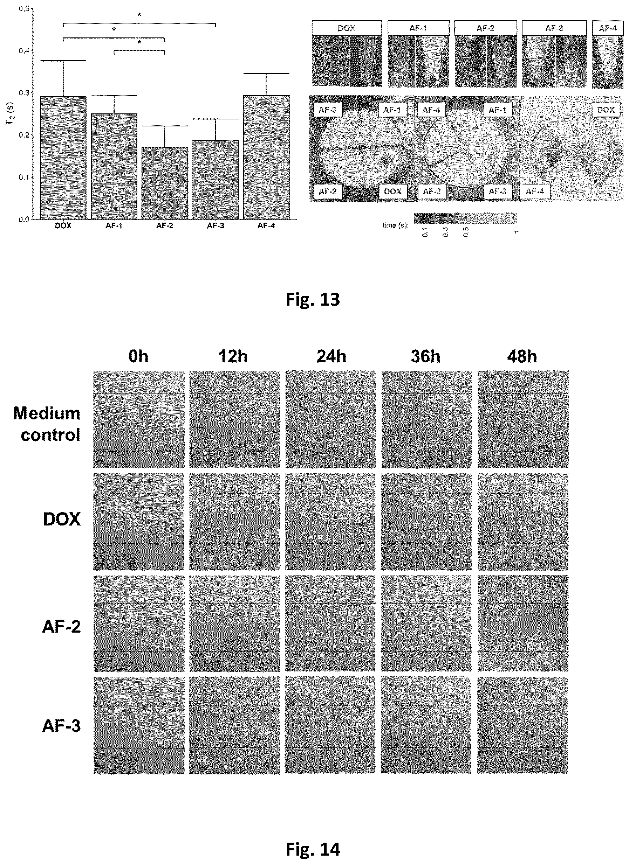

**Figure 13** shows the T2 relaxation times of Hs578T polyspheroids per active formulation tested. MRI was done in day 19 of the TNBC polyspheroid growth study. Images were taken on Eppendorfs and in compartmentalized petri dishes. * denotes a significance of at least $p < 0.05$. MRI contrast enhancement effect was observed in magnetically-labelled spheroid cells. In Figure 13A images were obtained using a preclinical 3 T MRI scanner under T1 and T2 acquisition modes using fast spin-echo (FSE) sequences for the treated polyspheroids. Using the ImageJ with MRI-T2 calculation plugin, it was obtained the graphic represented in Figure 13B for the T2 values (s, at 3 T 37 °C) of the different treatments applied. Results are presented as mean $\pm$ SD of at least three independent experiments. Results show a reduction of the transversal relaxation time of the cells in the polyspheroids due to the internalized iron. The functionalized nanoparticles AF2 (EBR85), AF3 (EBR86), and AF4 (EBBR89), all containing magnetite nanoparticles in the organic matrix, showed an enhancement of the T2-MRI contrast when internalized in Hs-578T polyspheroids, being the AF2 (EBR85) formulation the one displaying the lower T2 value.

**Figure 14** shows optical images to highlight the effect of mSLNs on the cell migration properties of Hs-578T cells after 0, 6, 24, 36 and 48h of incubation. The effect of the functionalized mSLNs AF-2 (EBR85, T-sensitive, +DOX) and AF-3 (EBR89, T-insensitive, -DOX) and reference compound (DOX) on the cell migration capability of Hs-578T cells was assessed through the wound-healing assay. mSLNs (with and without DOX) at the $IC_{50}$ DOX concentration and equivalent mSLNs were used in the experiment. EBR89 (without DOX) was selected to study the effect of the nanoparticle nature on this tumorigenic feature and evaluate its toxicity. Cell migration and invasion are two hallmarks of cancer correlated to cancer progression and metastasis which correspond to a worse cancer prognosis. Thus, **Figure 14** shows the progression of wound closure after different treatments with mSLNs. On the one hand, the non-treated cells (only received DMEM medium) showed a significant capacity to migrate through the inserted wound overtime. On the other, cells treated with fully functionalized mSLNs without DOX (EBR89) demonstrated to have a similar migratory profile as non-treated cells, showing 100% of wound closure after 48h of treatment. On the contrary, treatment with the DOX-loaded functionalized mSLNs (EBR85) at the $IC_{50}$ demonstrated to be effective on the reduction of the wound-healing capacity, with a significant decrease of cell migration compared to the control group (non-treated cells). The cells treated with the reference compound (DOX) showed a very low migration capability 48h post-treatment.

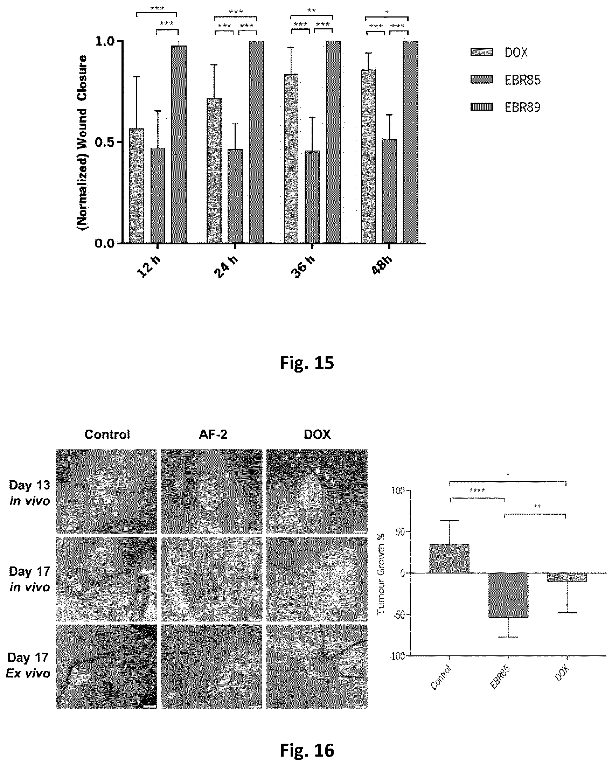

**Figure 15** shows the graphical representation of wound closure percentage (cell migration) relative to control group (DMEM medium). Results are presented as mean $\pm$ SD of at least three independent experiments. Treatment with the DOX-loaded functionalized mSLNs (EBR85) at the $IC_{50}$ demonstrated to be effective on the reduction of the wound-healing capacity, with a significant decrease of cell migration to approximately 45% compared to the control group (non-treated cells).

**Figure 16** shows the effect of the T-sensitive and DOX-loaded mSLNs (AF-2 = EBR85) nanoparticles and free DOX on Hs578t tumor growth in an *in ovo* model. (A) *In ovo* pictures at day 13 of embryo development and *in ovo* and *ex ovo* pictures at day 17; representative images were taken at 20x in a Zeiss stereomicroscope. (B) Graphical representation of tumor growth. Data was analyzed by one-way ANOVA and the results are representative for the mean of at least 15 eggs per group;***p<0.0001 *p<0.05. Hs578t cells were used to establish a TNBC chicken egg model. Fertilized eggs were incubated for 9 days and, at this time, the chicken embryo is surrounded by the CAM which provides nutrients for its development. At day 13 of development, the tumor mass could be visualized and the resulting tumors were treated with 2xIC50 EBR85-functionalized nanoparticles (corresponding to DOX concentration). The same concentration of DOX was used for the group treated with DOX alone. Also, a control group treated with DMEM medium was used to evaluate tumorgrowth. Both EBR85 and DOX revealed no toxicity to the chick embryo throughout the experiment, being observed a high survival percentage and with no signs of inflammation. As observed in **Figure 16,** a significant reduction on tumor size could be appreciated for the EBR85-treated group, comparing to the control and DOX-treated groups. Although the DOX-treated group demonstrated to have the capability to reduce tumor progression, the EBR85 proved to have the most effective in vivo profile, since treatment caused a regression in the tumors' area of approximately 53% while DOX-treatment caused a reduction of only 11%.

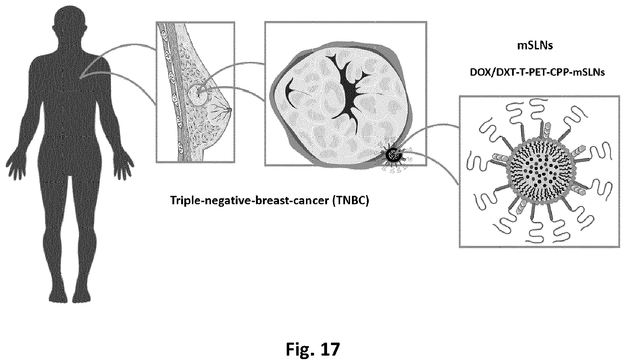

**Figure 17** shows a visual concept note highlighting the drug-loaded and functionalized mSLN and its application to treat triple-negative-breast-cancer.

In an embodiment, magnetic solid lipid nanoparticles (mSLNs) preparation was performed. A melt emulsification method was used for the preparation of mSLNs. Typically, in a glass vial crushed Carnauba wax added to a nanoparticle magnetite-oleate chloroform solution prepared following standard coprecipitation protocols (0 or 50 mgFe/100 mgwax). To this solution, different drugs (0 or 20 $mg_{drug}$ per 100 $mg_{wax}$), DiD (0.125mg per 100 $mg_{wax}$), surfactants (17.5% in total regarding the wax mass in a typical synthesis) and water (2.25 ml per 100 $mg_{wax}$) were added. Then the mixture was heated to melt the wax. The samples were ultrasonicated for 2 min at 25% power at 20-second working intervals, whilst heating with a heating gun and avoiding water boil. After, the samples were immersed in icy-water to solidify the preparations directly after the sonication, and once cold, they were centrifuged to remove large wax aggregates (3000 rpm, 10 min). The supernatant was stored at 4°C or freeze dried (in the presence of sucrose; 1% w/v) as cryoprotectant and stored at -40 ºC.

In an embodiment, mSLNs characterization was performed. The magnetic solid lipid nanoparticles of the present disclosure were characterized for their ζpot and hydrodynamic diameters values using a Horiba Scientific Nanopartica SZ-100 dynamic light scattering (DLS) equipment. A JEOL JEM-2100 microscope operating at 200 kV accelerating voltage was used for transmission electron microscopy images (TEM). Iron and platinum concentrations were determined through inductively coupled plasma-optical emission spectroscopy (ICP-OES) using an ICPE-9000 Multitype ICP Emission Spectrometer from Shimadzu. Fluorescence spectroscopy was carried out in a FluoroMax-4 Compact Spectrofluorometer.

In an embodiment, drug loading and release of the magnetic solid lipid nanoparticles of the present disclosure were determined by HPLC using an Agilent 1290 Infinity II LC system equipped with fluorescence (excitation wavelength of 488 nm and emission wavelength of 560 nm) and absorbance detectors to quantify drug concentrations. The encapsulation within the magnetic solid lipid nanoparticles of the present disclosure were calculated indirectly taking into account the initial mass of drug used. The drug release was studied using the Pur-A-LyzerTM Mega Dialysis Kit. The HPLC method used solvent A = (H2O + 0.1 % TFA) and solvent B = (ACN + 0.1 % TFA) to determine the drug's retention time and concentrations for each sample. Starting from 100 % A, the ratio of B was increased steadily for 10 min until 25/75 % of A/B was reached. This mixture was maintained for 5 minutes and then the degree of A was increased for another 5 min to 100 % of A. 100 % of A was further maintained for 5 minutes.

In an embodiment, the magnetic properties of the formulations as heat effectors were investigated using a nB nanoScale Biomagnetics DM-100 instrument. The specific absorption rates (SAR) were calculated through the initial slope method using {SAR = C. c-1.deltaT.deltat-1} as formula, where C is the specific heat capacity of the medium (assumed equal to water, C water = 4185 J L-1K-1), c is the iron concentration (g L-1) of the magnetic material in solution, and $\Delta T.\Delta t$-1 K.s-1 is the initial slope of the T versus time curve from the heating curves. Magnetically induced drug release experiments were carried out in a Magnetherm instrument from Nanotherics.

In an embodiment, magnetic resonance imaging (MRI) was performed. MR imaging was performed in a 3.0 T horizontal bore MR Solutions Benchtop MRI system equipped with 48 G/cm actively shielded gradients. To image the samples, a 56-mm diameter quadrature birdcage coil was used in transmit/receive mode. For phantom imaging, samples at different concentrations (0-100 µM Fe) were prepared in milliQ water (300 µL) and pipetted into a custom printed PLA wellplate. All MR images of phantoms were acquired with an image matrix 256x252, FOV 60x60 mm, 3 slices with a slice thicken ss of 1 mm and 0.5 mm slice gap. For T 2-weighted imaging, fast spin echo (FSE) sequences with the following parameters were used: TE = 14ms, TR = 3000 ms, NA = 3, AT = 9m 27s.

In an embodiment, phenotypic in vitro evaluation was performed to determine the toxicity of the controls without drugs, the formulations were assumed to be loaded with the same drug-quantity, as to calculate the toxicity contribution of all other components in the nanoformulations.

In an embodiment, 2D- cell culture was prepared. Hs-578T and MBA-MB-231 TNBC cell lines (ATCC HTB-126 and ATCC Htb-26, respectively) were grown in Dulbecco's Modified Eagle's Medium (DMEM) supplemented with 10% foetal bovine serum and 1% penicillin/streptomycin. THP-1 peripheral blood monocytes (ATCC TIB-202) were grown in Gibco Roswell Park Memorial Institute (RPMI) medium supplemented with 10% foetal bovine serum and 1% penicillin/streptomycin. All cell lines were incubated at 37 °C in a 5% CO2 atmosphere.

In an embodiment, cell viability was assessed. The Resazurine assay was used to calculate the cell viability. The assay is based on the reduction by living cells of the oxidized blue dye to a pink fluorescent resorufin product. After 24, 48 and 72 h of exposure to the different nanoformulations, the cells were photographed with an optical microscope and resazurine was added (1x) and incubated with the cells for four additional hours. The fluorescence of each condition was then read with an Biotek Synergy H1 plate reader, and compared to the controls using the formula {*Viability* = $\frac{Treated\ cells-blank}{Untreated\ cells-blank} \times 100$ }. The experiments were repeated at least three times in duplicate. For each, the inhibitory concentration 50 (IC50) of each formulation was calculated using the DRC R-package. The concentration (IC50) is a measure of the potency of a substance in inhibiting a specific biochemical or biological function, such as its viability. The activity gain (AG) for the different formulations was calculated as
$$AG_F = 100 \times \frac{IC_{50}^{free\,DOX} - IC_{50}^{mSLNP}}{IC_{50}^{free\,DOX}}$$

, whilst the AG of the magnetic hyperthermia event was calculated as
$$AG_{MH} = 100 \times \frac{IC_{50}^{noHT} - IC_{50}^{HT}}{IC_{50}^{noHT}}$$
. Cell viability experiments under MH stimulation were performed in a Magnetherm instrument from Nanotherics equipped with a Live Cell alternating magnetic field exposure system at 20 mT and 88 nF.

In an embodiment, 3D spheroids were prepared. In a 96-well plate was a hot and sterile solution of 1% agarose added (100 $\mu$L) and left to cool for 30 min at 4 $\underline{o}$C. Then, the plate was tempered and 20000 cancer cells added to each well in 120 $\mu$L of medium. The plate was centrifuged at 800 G during 30 min to promote the formation of the spheroid. The Spheroids were incubated for one week with a medium change every 3 days, until a small dense Spheroid was formed. These spheroids were used in confocal microscopy, or to form poly-spheroids. To do the latter, the spheroids were changed from the 96-well plate to a compartmentalized (in four) Petri dish pre-treated with 1% agarose following the previous protocol. After cooldown, 4 spheroids were seeded together per compartment in 300 $\mu$L of medium. After another week of incubation and medium changes, the compact poly-spheroid formed and was used for the spheroid growth assay.

In an embodiment, growth assay was performed. In day 1, 3 and 5 of the assay, the medium of the petri-dish was changed and 2$\mu$M of free doxorubicin or doxorubicin-loaded mSLNs was added (roughly double the 2D-in vitro IC50 found). The polyspheroids were then treated for 1h with an alternating magnetic field at 20 mT and 88 nF. After every treatment, the spheroids were incubated at 37 °C in a 5% $CO_2$ atmosphere. On day 8, 10, 12, 15, 17 and 19, the mediums were renewed without adulterations. The polyspheroids were monitored every 2 days before treatments or medium change, using an inverted phase-contrast microscope (Nikon Eclipse TS100, Nikon Corporation, Tokyo, Japan) connected to a digital camera (Nikon, DS-Fi1). All conditions were done in quadruplicate and repeated at least two times. The images were analyzed with image J and R statistical language, using R-packages imager and EBImage, to determine the area of each polyspheroids treated with each condition for every day analyzed. The growth of the polyspheroids were normalized in regards to their own initial area at the start of the experiment, and calculated as { $Growth =$
$$100 \times \frac{Area - Area^{day1}}{Area^{day1}}$$ }. The daily growth was calculated as
$$Growth^{daily} = \frac{\sum Growth}{19\,days}$$
. In day 19, the samples were first imaged by magnetic resonance, and then dissolved in hydrochloric acid for their iron determination by ICP.

In an embodiment, confocal microscopy was performed. 2D-cells and 3D-spheroids were seeded in plates and treated with the formulations (**Figures 6 and 5, respectively**). Then their DNA were stained with Hoechst-33342 reagent one hour prior the analysis. DiO was used as cell dye using a solution of 5$\mu$M in media from a stock of 1mM in ethanol or DMSO (as https://promocell.com/wp-content/uploads/2018/01/Di_Labeling_Protocol.pdf). Confocal microscopy images were obtained with a Carl Zeiss inverted microscope attached to the LSM 780 confocal system (software; ZEN 2013).

In an embodiment, magnetic solid lipid nanoparticles were prepared and characterized. The family of 1,2-Dipalmitoyl-glycero-3-phosphoethanolamine (DPPE) derivatives were synthesized and characterized using co-surfactants on the nanoformulations. These included phospholipids (PL):

1) PL-3: with a thermo-sensible bond between the PL and the PEG-polymer (Diels-Alder bond).
2) PL-4: with a thermo-insensible bond between the PL and the PEG-polymer (amide bond)
3) PL-6: with a thermo-insensible bond between the PL and a cell penetrating peptide (CPP; cys-TAT)
4) PL-7: with a thermo-sensible bond between the PL and a fluorescent coumarin moiety (Diels-Alder bond).

In an embodiment, magnetic solid lipid nanoparticles loaded with magnetite nanoparticles and chemotherapeutic compounds were prepared. On their surface, cell-penetrating power of PL-6 and the bioactivity of the PEG polymer in PL-3 were combined. The PEG moiety protects and sterically hinders the CPP in PL-6 until activated by the AMF. As the PEG ligand in PL-3 is attached through a thermo-reversible Dials-Alder equilibrium, the heat generated by the magnetite when under an AMF is capable of breaking the bond (**Figure 9A**). This PEG-detachment exposes the CPP in PL-6, promoting an effective translocation of the formulation to nearby cells. Therefore, when an AMF is locally supplied at the tumour site, an increase in the drug-related toxicity is expected as consequence of the magnetic hyperthermia-triggered targeting efficiency, which promotes the cell internalization of the mSLNs in the tumor cells and the local release of the encapsulated drug, without the use of any specific membrane receptors as targeting ligands.

The synthesis of the magnetic solid lipid nanoparticles with the modified PL (DPPEM) was first optimized through the study of the best combination of surfactant types and quantities to form the magnetic solid lipid nanoparticles (DPPE, DPPC and Tween 80; 8.75, 12.5% and 20% w/w wax). These were to produce magnetic solid lipid nanoparticles of sizes between 100 and 200 nm, and sufficiently loaded with magnetite to be able to break PL-3 under an AMF. We also optimized the chemotherapeutic drug type to be loaded within the system to obtain the highest activity (Doxorubicin, Docetaxel or Oxaliplatin). Magnetic solid lipid nanoparticles prepared when using 12.5 % of Tween 80 (w/w with wax) in combination with 3.75 % of PL-3 and 1.25 % of PL-6 as surfactants, loaded with 20 % of Doxorubicin (DOX) and 50 % of magnetite displayed the best results for the synthesis of the envisioned active formulation (AF-2, >> **in Figure 9C**).

The following magnetic solid lipid nanoparticles were prepared as controls:

AF-1; without DPPEM as a control to study the effect of the modified PL

AF-3; with the thermo-insensible PL-4 instead of PL-3 as a control to study the effect of the thermo-sensible bond

AF-4; without DOX as a control to study the drug effect

AF-5; only with PL-6 as a positive control for confocal microscopy

AF-6; without magnetite as a control to study the effect of the magnetic hyperthermia

The sizes measured by TEM and DLS were in all cases in the range of ~100 to 200 nm, and their DOX encapsulation within the system was efficient. The magnetite encapsulation was lower than that of DOX, although the concentration of magnetite within the stock solution was able to at least raise the macroscopic solutions' temperature over 50 $^\circ$C.

**Figure 9A** shows the conceptualization of the magnetic solid lipid nanoparticles prepared and the activation process through magnetic hyperthermia. **Figure 9B** shows TEM images of the magnetite loaded formulations. **Figure 9C** shows the active formulations (AF) prepared (with an additional 12.5 mg of Tween 80 in 2.25 mL of water) and their properties, including their doxorubicin encapsulation efficiency (DOX. EE.), as well as magnetic hyperthermia and MRI contrast enhancement efficiencies (SAR and relaxivity, respectively). The envisioned active formulation was highlighted with double arrows ">>".

In an embodiment, to test the Diels-Alder equilibrium as an effective thermo-sensible mechanism for the activation of the formulations, PL-3's equilibrium in a molecular level was evaluated using DMSO as solvent in NMR spectroscopy (**Figure 2C**). The evaluation was performed for 72 hours at 85 °C and analyzed the PEG-reagent's maleimide signal using as reference other constant signals through-out the equilibrium to determine its state. Results showed that after 2 hours at 85 °C, over 50 % of the Diels-Alder bond in PL-3 could reverse to its initial products, and after 24h, the conversion was almost complete. Additionally, it was also determined if this conversion could happen in water and triggered by the heat generated in the magnetic hyperthermia event (mHT), by synthesizing, purifying and characterizing a family of magnetic solid lipid nanoparticles using fluorescent PL-7 in combination to Tween 80 as surfactants (without DOX or any other PL) (**Figure 8**). The idea was to use this family of magnetic solid lipid nanoparticles to measure their relative change of fluorescence caused by the coumarin-moiety break-off after the mHT, associated with the retro Diels-Alder. The sharp increase in fluorescence showed after the mHT confirms that the retro Dials-Alder reaction was triggered in water by mHT effect. Altogether, these results indicate that the formulations have the capability to be activated by the external stimulus (AMF) in the biological media.

In an embodiment, further studies were carried out to test the functionalized magnetic solid lipid nanoparticles mSLNs DOX-transport and -release capabilities. Using HPLC to quantify the drug, AF-2 (Active mSLNs, with PL-3 and PL-6) and AF-1 (control magnetic solid lipid nanoparticles, without PLs) 60 hours cumulative DOX release profiles were compared (**Figure 10, upper**). Additionally, the effect of the mHT on the DOX release was also studied at initial stages (**Figure 10, lower**). It was observed that the formulations' passive release of DOX, which best fitted logarithmic/sigmoidal models, was different between formulations, as AF-2 managed to release a higher amount of DOX compared to AF-1 at the experimental end point (45 vs 35 % of total loaded DOX, respectively). However, during the initial 4 h of passive release, AF-1 released a higher amount of DOX per hour than AF-2 (6.8 vs 5.4 % per hour). When an AMF was applied, the initial release rate increased in both cases to ~8.8 % of total DOX release per hour. Hence, compared to their own passive rate, DOX release increased due to mHT was higher in AF-2 than in AF-1 (39 % vs 23 %, respectively). These results were further supported by TEM characterization of the formulations. Images were taken before and after the mHT to also determine the integrity of the formulations after the application of the AMF (**Figure 11**). As the figures showed, the morphology of the formulations was affected and part of the lipidic and magnetic contents burst-out of the formulations, which may be related to the increase in its DOX release rate as determined by HPLC.

In an embodiment, 2D-cell viability studies were performed. The active family of magnetic solid lipid nanoparticles (AF-1 to AF-4) were then evaluated in 2D-in vitro cell viability assays in TNBC (Hs-578t) and in healthy monocytes (THP-1) cells, and compared to the same concentrations of free DOX (**Figure 4**).

The drug-unloaded formulation (loaded with and without magnetite) was not toxic at the maximum concentrations

tested (100 $\mu$M, relative to the same quantity of formulations loaded with DOX; **Figure 7C**). However, when loaded with DOX, the formulations with functionalized surfactants displayed greater activity gain (AG) than the free DOX in the TNBC cell line for all time points tested (**Figure 4A**). At the maximum time-point of 72h, AF-2 (Active mSLNs, with PL-3 and PL-6) decreased the IC50 of DOX from 1.60 to 1.06 $\mu$ M (activity gain with respect to DOX, $AG_{DOX}$ = 34%), whilst AF-1 only to 1.37 $\mu$ M (control mSLNs, without modified PLs; $AG_{DOX}$ = 14 %). AF-3 (control mSLNs, with thermo-insensible PL-4 and PL-6) displayed an overall slightly smaller effect than AF-2, although greater than AF-1 and free DOX.

In an embodiment, the activity of DOX was found to be all-around more pronounced in THP-1 (**Figure 4B**). However, opposite to what was observed for the TNBC, DOX delivered from the magnetic solid lipid nanoparticles was less toxic than that of the same concentration of free DOX at all time points. This was especially relevant for the formulations with PEG-ligands after shorter incubation times. AF-2 displayed an activity gain of -265 % compared to free DOX after 24 h (active magnetic solid lipid nanoparticles with **PL-3** and **PL-6**), whilst AF-3 of -198 % (control magnetic solid lipid nanoparticles, with thermo-insensible **PL-4** and **PL-6**). When incubating the formulations for 48 h, the marked reduction of activity was still appreciable, although it was not as strong as that measured at the 24 h time-point. For example, AF-2 showed a decrease in activity gain of 131 % ($AG^{DOX}$ = -131 %). After 72 h, the effect totally disappeared and their activities were found to be in the range of the unfunctionalized formulation (AF-1).

In an embodiment, confocal microscopy studies were performed. TNBC cells were incubated with the nano-formulations loaded with DiD but without DOX and used confocal microscopy to analyze their cellular-uptake. The active formulations were incubated for 5 hours after the application, or not, of the AMF. Here, we compared AF-2 (active mSLNs with PL-3 and PL-6) with AF-1 (control magnetic solid lipid nanoparticles without modified PL and used as negative control) and AF-5 (control magnetic solid lipid nanoparticles only with the PL-6; with the exposed CPP-moiety to promote cellular internalization and used as positive control). AF-2 and AF-1 were barely internalized without the application of the AMF as it can be seen in **Figure 6A first column, first two rows.** Yet when the AMF was applied for 1 hour, AF-2 fluorescence drastically increased within the cells. The intensity of the DiD signal in this magnetic environment resembled more similarly that of AF-5, which with and without mHT had managed to clearly be internalized more by the cultured cells (**Figure 6A third row and B**). When the experiment was repeated with incubations of 24 hours, the DiD signals were found equally saturated for all conditions.

**Figure 6A** shows confocal microscopy images (x40 objective) of Hs-578T cells incubated with the formulations loaded with the fluorophore DiD, for 5 h at 37 $\underline{o}$C (no HT, first column pair), and with the application of an AMF for 1h and 4 h of posterior incubation at 37 $\underline{o}$C (1h HT, second column pair). The first column of each pair is the red channel corresponding to DiD ($\lambda_{exc.}$ = 633 nm, $\lambda_{em.}$ = 687 nm), whilst the second column of each pair is the superposition and merge of all channels; DiD's red for the identification of the mSLNs, Hoechst-33342's blue for the visualization of the cell's nucleus ($\lambda_{exc.}$ = 405 nm, $\lambda_{em.}$ = 434 nm) and DiO's green for the identification of the cell ($\lambda_{exc.}$ = 488 nm, $\lambda_{em.}$ = 525 nm). **Figure 6B** shows the average red channel fluorescence quantification per cell, from A.

In an embodiment, 3D-TNBC Polyspheroid growth studies were performed. The formulations were tested in a more biologically complex model using sufficiently big polyspheroids to be detected in MRI. TNBC polyspheroids were treated three times with a dual treatment consisting in the application of 1h AMF and 2 $\mu$M of the different nanoformulations and controls, and incubated for 19 days (Figure 5, A). The effect each condition had on the polyshperoids growth was monitored and normalized to the area each polyspheroid had on day 1. This way we compared AF-2, (active mSLNs with PL-3 and PL-6) with all other controls, which included free DOX, AF-1 (without modified PL), AF-3 (with PL-3 and PL-6, thermo-insensible PEG control) and AF-4 (without DOX, mHT control).

**Figure 5A** shows the evolution of Hs-578T polyspheroid growth under different treatment conditions. Dual treatment of incubations with the mSLNs (2 $\mu$M, relative to DOX concentration within the mSLNs) and exposure to an AMF for 1h was administered in days 1, 3 and 5. Each polyspheroids' initial volume (day 1) was used as individual reference from which its posterior growth was calculated. Four polyspheroids were used per condition and experiment. **Figure 5B** shows the mean daily polyspheroids growth per condition. Figure 5C shows the confocal images of the top of a polyspheroid treated with Hoechst-33342 dye (I, blue), AF-2 (II, red), DiO (III, green).

The polyspheroids continued to grow during the first week of treatment with free DOX and the mHT control (AF-4). AF-1 initially managed to reduce their growth but they recovered in size in posterior weeks. The active AF-2 and the control AF-3 - however- reduced polyspheroid growth initially and prevented them from recovering their original size afterwards **(Figure 5A).** This effect on the TNBC polyspheroids was not observed for treatments only with free DOX or only with the mHT event (AF-4), where the tumour mass in average grew daily.

In an embodiment, the active AF-2 was visualized using confocal microscopy after incubation with the Hs-578T polyspheroids. AF-2 was found distributed all over the top area of the polyspheroid after 24h incubations (Figure 5C). The last day of the experiment, the polyspheroids were also visualized by MRI and studied as potential contrast agents (**Figure 13**).

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The embodiments described above are combinable.

The following claims further set out particular embodiments of the disclosure.

The authors would like to acknowledge the financial support from The Foundation for Science and Technology "Fundação para a Ciência e a Tecnologia" and the ERDF (European Regional Development Fund) by NORTE 2020 (2014-2020 North Portugal Regional Operational Program) through the project NORTE-01-0145-FEDER-031142 (MAGTARGETON - Local specific treatment of triple-negative-breast-cancer through externally triggered target-less drug carriers).

## References

1) Nanopartículas lipídicas solidas a base de cera de carnauba com potencial aplicação em hipertermia magnetica e imagen por ressonancia magnetica. BR 10 2016 016 795 7.

2) Lorena García-Hevia, Íñigo Casafont, Jessica Oliveira, Nuria Terán, Mónica L. Fanarraga, Juan Gallo and Manuel Bañobre-López. Magnetic lipid nanovehicles synergize the controlled thermal release of chemotherapeutics with magnetic ablation while enabling non-invasive monitoring by MRI for melanoma theranostics. Bioactive Materials 8, 153-164 (2022). https://doi.org/10.1016/j.bioactmat.2021.06.009

3) Lourdes Valdivia, Lorena García-Hevia, Manuel Bañobre-López, Juan Gallo, Rafael Valiente, Mónica Lopez-Fanarraga. "Solid-lipid particles for lung metastasis treatment". Pharmaceutics 2021 Jan 13;13(1):93. doi: 10.3390/pharmaceutics13010093.

4) Julia Jiménez-López, Lorena García-Hevia, Consolacion Melguizo, Jose Carlos Prados, Manuel BañobreLópez and Juan Gallo. Evaluation of novel magnetic wax nanocomposite vehicles as cancer combinatorial therapy agents. Pharmaceutics 12(637):4715-330. doi: 10.3390/pharmaceutics12070637.

5) Cátia Rocha, Milene Silva, Manuel Bañobre-López and Juan Gallo. (Para)magnetic hybrid nanocomposites for dual MRI detection and treatment of solid tumours. Chem. Commun., 2020, 56, 8695. doi: 10.1039/d0cc03020a.

6) Carolina L. de Moura, Juan Gallo, Lorena García-Hevia, Otilia D. L. Pessoa, Nagila M. P. S. Ricardo and Manuel Bañobre-López. "Magnetic Hybrid Wax Nanocomposites as Externally Controlled Theranostic Vehicles: High MRI Enhancement and Synergistic Magnetically Assisted Thermo/Chemo Therapy". Chem. Eur. J. 2020, 26, 4531 - 4538. COVER FEATURE 20/2020. https://doi.org/10.1002/chem.201904709; https://chemistry-urope.onlinelibrary.wiley.com/doi/epdf/10.1002/chem.202000810

7) International Journal of Cosmetic Science, 2011, 33, 312-321.

8) Langmuir 2017, 33, 1051-1059.

9) Angewandte Chemie International Edition 46(32):6126-31).

10) Chemical Engineering Journal, 380, 2020, 122458

11) Mol Pharm. 2014 Dec 1; 11(12): 4425-4436.

12) Mol. Ther. Acids 2016, 5 (7), e340.

## Claims

1. A magnetic solid lipid particle comprising:

an encapsulated core, wherein said core comprises thermal effectors and/or MRI contrast enhancers, a surfactant for stabilizing the magnetic solid lipid particle in aqueous solution, and an active ingredient;

wherein the thermal effectors and/or MRI contrast enhancers are magnetic nanoparticles;
a first modified phospholipid comprising a poly-ethylene glycol molecule - PEG - bonded onto the surface of the encapsulated core and; wherein said PEG modified phospholipid is temperature responsive; and
a second modified phospholipid comprising a cell penetrating peptide - TAT - bonded onto the encapsulated core and entrapped by the PEG ligand;
wherein a remotely applied electromagnetic stimulus causes magnetic hyperthermia wherein the temperature-responsive PEG releases and exposes the phospholipid cell penetrating peptide to locally target cells and allow subsequent release of the active ingredient in to the target cells.

2. The lipid particle according to any of the previous claims, wherein the surfactant is Tween 80, polysorbate 80, sorbitol, or mixtures thereof.

3. The lipid particle according to any of the previous claims, wherein the modified phospholipid is selected from phospholipid T-sensitive PEG, phospholipid cell penetrating peptide, or mixtures thereof.

4. The lipid particle according to the previous claim, wherein the core is a wax; preferably carnauba wax, bee wax, or mixtures thereof.

5. The lipid particle according to any of the previous claims, wherein the thermal effectors magnetic nanoparticles are magnetite nanoparticles, iron oxide nanoparticles, or mixtures thereof.

6. The lipid particle according to any of the previous claims, wherein the MRI contrast enhancers magnetic nanoparticles are magnetite nanoparticles, iron oxide nanoparticles, or mixtures thereof.

7. The lipid particle according to any of the previous claims, wherein the PEG molecular weight ranges from 2000 Da to 10000 Da, preferably 5000 Da.

8. The lipid particle according to any of the previous claims, wherein the active ingredient is an anticancer drug, an antimalaria drug, an antifungal drug, an antibiotic, an immune-modulator, a calcium blocker, or a vitamin.

9. The lipid particle according to any of the previous claims, wherein the anticancer drug is docetaxel, oxaliplatin, doxorubicin, 5-Fluorouracil, Cisplatin, Bortezomib, or mixtures thereof.

10. Pharmaceutical composition comprising the lipid particle according to any of the previous claims, for use in medicine or as a medicament.

11. The composition according to the previous claim, for use as a chemotherapy agent, as a thermotherapy agent, as thermochematherapeutic agent and as a theranostic agent.

12. The composition according to any of the previous claims 10-11, for use in the treatment of a solid cancer.

13. The composition according to any of the previous claims 10-12, wherein the solid cancer is breast cancer.

14. The composition according to any of the previous claims 10-13, wherein the breast cancer is triple negative breast cancer.

15. Method of preparing the magnetic solid lipid particle according to any of the previous claims 1-9 comprising:

   melting wax;
   adding a surfactant, modified phospholipids, a magnetic nanoparticles and an active ingredient to the melted wax to form a suspension;
   wherein the modified phospholipids comprises:

      a first modified phospholipid comprising a poly-ethylene glycol molecule - PEG - bonded onto the surface of the encapsulated core and; wherein said PEG modified phospholipid is temperature responsive; and
      a second modified phospholipid comprising a cell penetrating peptide - TAT - bonded onto the encapsulated core and entrapped by the PEG ligand;

subjecting the obtained suspension to ultrasonication;
placing the ultrasonicated suspension in a cold bath to solidifying lipid nanoparticles in the suspension;
centrifugating the suspension to obtain supernatant comprising the lipid nanoparticles.

Fig. 1

**Fig. 2**

**Fig. 3**

Fig. 2

**Fig. 2**

**Fig. 3**

Fig. 4

Fig. 5

**Fig. 4**

**Fig. 4**

**Fig. 5**

**Fig. 6**

**Fig. 7**

**Fig. 8**

Fig. 7

**Fig. 7**

| | Composition | | | Characteristics | | | | Hyperthermia | | | Fluorescence | |
|---|---|---|---|---|---|---|---|---|---|---|---|---|
| | Wax | Magnetite | PL 7 | Size (DLS) | Size (TEM) | Surface charge | Iron cc. | max ΔTemp. | SAR | ILP | before HT. | after HT. |
| | mg | mg | mg | nm | nm | mV | mg/mL | °C | W/g | kA/m | % | % |
| FF-1 | 100 | ● | 50 | ⇞ 3.75 | 105 ± 28 | 79 ± 31 | -67 | 0.73 | 21 | 216 | 0.98 | 109 | 168 |
| FF-2 | 100 | ○ | 0 | ⇞ 3.75 | 62 ± 7 | NA | -73 | 0 | 2 | NA | NA | 100 | 100 |
| FF-3 | 100 | ◐ | 50 | 0 | 171 ± 4 | 114 ± 49 | -75 | 1.86 | 65 | 269 | 1.22 | -31 | -31 |

**Fig. 8**

Fig. 8

**Fig. 9**

**Fig. 10**

**Fig. 11**

**Fig. 12**

**Fig. 13**

**Fig. 14**

**Fig. 15**

**Fig. 16**

Fig. 17

Europäisches
Patentamt

European
Patent Office

Office européen
des brevets

# EUROPEAN SEARCH REPORT

Application Number

EP 22 16 1429

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | GARCÍA-HEVIA LORENA ET AL: "Magnetic lipid nanovehicles synergize the controlled thermal release of chemotherapeutics with magnetic ablation while enabling non-invasive monitoring by MRI for melanoma theranostics", BIOACTIVE MATERIALS, vol. 8, 17 June 2021 (2021-06-17), pages 153-164, XP055956809, ISSN: 2452-199X, DOI: 10.1016/j.bioactmat.2021.06.009 * whole document, especially Figure 1A * | 1-15 | INV. A61K47/69 A61K41/00 A61P35/00 |
| A | CN 107 019 801 A (UNIV SICHUAN) 8 August 2017 (2017-08-08) * paragraph [0094]; claims 1-10 * | 1-15 | |
| A | KIM CHANG HYUN ET AL: "Surface modification of lipid-based nanocarriers for cancer cell-specific drug targeting", JOURNAL OF PHARMACEUTICAL INVESTIGATION, SPRINGER SINGAPORE, SINGAPORE, vol. 47, no. 3, 17 April 2017 (2017-04-17) , pages 203-227, XP036228613, ISSN: 2093-5552, DOI: 10.1007/S40005-017-0329-5 [retrieved on 2017-04-17] * whole document and especially pages 205-206 section "SLNs and NLCs"; Figures 2, 3; pages 214-215, section "CPPs and CPHPs for enhanced intracellular delivery"; Tables 2, 3 * | 1-15 | TECHNICAL FIELDS SEARCHED (IPC) A61K |
| A | US 2020/368161 A1 (LO YU-LI [TW]) 26 November 2020 (2020-11-26) * claims 1, 2, 8, 10, 11, 16, 19 * | 1-15 | |

-/--

The present search report has been drawn up for all claims

| Place of search | Date of completion of the search | Examiner |
|---|---|---|
| The Hague | 2 September 2022 | Birikaki, Lemonia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 16 1429

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HAMMAD MOHANED ET AL: "On-command controlled drug release by diels-Alder reaction using Bi-magnetic core/shell nano-carriers", COLLOIDS AND SURFACES B: BIOINTERFACES, ELSEVIER AMSTERDAM, NL, vol. 150, 5 November 2016 (2016-11-05), pages 15-22, XP029888946, ISSN: 0927-7765, DOI: 10.1016/J.COLSURFB.2016.11.005 * abstract * ----- | 1-15 | |
| A | MING-HUANG HSU ET AL: "Iron-oxide embedded solid lipid nanoparticles for magnetically controlled heating and drug delivery", BIOMEDICAL MICRODEVICES, KLUWER ACADEMIC PUBLISHERS, BO, vol. 10, no. 6, 17 June 2008 (2008-06-17), pages 785-793, XP019639769, ISSN: 1572-8781, DOI: 10.1007/S10544-008-9192-5 * whole document, and in particular Figure 1 and page 789, section "5 Results and discussion" * ----- | 1-15 | TECHNICAL FIELDS SEARCHED (IPC) |

The present search report has been drawn up for all claims

| Place of search | Date of completion of the search | Examiner |
|---|---|---|
| The Hague | 2 September 2022 | Birikaki, Lemonia |

EPO FORM 1503 03.82 (P04C01)

**page 2 of 2**

## ANNEX TO THE EUROPEAN SEARCH REPORT
## ON EUROPEAN PATENT APPLICATION NO.

EP 22 16 1429

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2022

| Patent document cited in search report | | Publication date | Patent family member(s) | | Publication date |
|---|---|---|---|---|---|
| CN 107019801 | A | 08-08-2017 | NONE | | |
| US 2020368161 | A1 | 26-11-2020 | TW | 202108127 A | 01-03-2021 |
| | | | US | 2020368161 A1 | 26-11-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- BR 1020160167957

**Non-patent literature cited in the description**

- **LORENA GARCÍA-HEVIA ; ÍÑIGO CASAFONT ; JESSICA OLIVEIRA ; NURIA TERÁN ; MÓNICA L. FANARRAGA ; JUAN GALLO ; MANUEL BAÑOBRE-LÓPEZ.** Magnetic lipid nanovehicles synergize the controlled thermal release of chemotherapeutics with magnetic ablation while enabling non-invasive monitoring by MRI for melanoma theranostics. *Bioactive Materials,* 2022, vol. 8, 153-164, https://doi.org/10.1016/j.bioactmat.2021.06.009
- **LOURDES VALDIVIA ; LORENA GARCÍA-HEVIA ; MANUEL BAÑOBRE-LÓPEZ ; JUAN GALLO ; RAFAEL VALIENTE ; MÓNICA LOPEZ-FANARRAGA.** Solid-lipid particles for lung metastasis treatment. *Pharmaceutics,* 13 January 2021, vol. 13 (1), 93
- **JULIA JIMÉNEZ-LÓPEZ ; LORENA GARCÍA-HEVIA ; CONSOLACION MELGUIZO ; JOSE CARLOS PRADOS ; MANUEL BAÑOBRELÓPEZ ; JUAN GALLO.** Evaluation of novel magnetic wax nanocomposite vehicles as cancer combinatorial therapy agents. *Pharmaceutics,* vol. 12 (637), 4715-330
- **CÁTIA ROCHA ; MILENE SILVA ; MANUEL BAÑOBRE-LÓPEZ ; JUAN GALLO.** Para)magnetic hybrid nanocomposites for dual MRI detection and treatment of solid tumours. *Chem. Commun.,* 2020, vol. 56, 8695
- **CAROLINA L. DE MOURA ; JUAN GALLO ; LORENA GARCÍA-HEVIA ; OTILIA D. L. PESSOA ; NAGILA M. P. S. RICARDO ; MANUEL BAÑOBRE-LÓPEZ.** Magnetic Hybrid Wax Nanocomposites as Externally Controlled Theranostic Vehicles: High MRI Enhancement and Synergistic Magnetically Assisted Thermo/Chemo Therapy. *Chem. Eur. J.,* 2020, vol. 26, 4531-4538
- *International Journal of Cosmetic Science,* 2011, vol. 33, 312-321
- *Langmuir,* 2017, vol. 33, 1051-1059
- *Angewandte Chemie International Edition,* vol. 46 (32), 6126-31
- *Chemical Engineering Journal,* 2020, vol. 380, 122458
- *Mol Pharm.,* 01 December 2014, vol. 11 (12), 4425-4436
- *Mol. Ther. Acids,* 2016, vol. 5 (7), e340